# EUROPEAN PATENT SPECIFICATION

(11) **EP 3 798 304 B1**
(45) Date of publication and mention of the grant of the patent: **11.12.2024**
(21) Application number: 19807027.8
(22) Date of filing: 19.04.2019
(51) Int. Cl.: C12N 15/00, A23L 33/10, A61K 35/76, A61P 31/04, C12N 7/01, C12N 15/31, C12Q 1/04, G01N 33/50, C12N 15/86

(54) **ANTIBACTERIAL PHAGE, TREATMENT COMPOSITION, DISINFECTANT, FOOD, KIT FOR IDENTIFYING BACTERIA, METHOD FOR PRODUCING TREATMENT COMPOSITION, METHOD FOR ELIMINATING BACTERIA, METHOD FOR IDENTIFYING BACTERIA, AND METHOD FOR TREATING ANIMALS**
ANTIBAKTERIELLE PHAGE, BEHANDLUNGSZUSAMMENSETZUNG, DESINFEKTIONSMITTEL, NAHRUNGSMITTEL, KIT ZUR IDENTIFIZIERUNG VON BAKTERIEN, VERFAHREN ZUR HERSTELLUNG EINER BEHANDLUNGSZUSAMMENSETZUNG, VERFAHREN ZUR BESEITIGUNG VON BAKTERIEN, VERFAHREN ZUR IDENTIFIZIERUNG VON BAKTERIEN UND VERFAHREN ZUR BEHANDLUNG VON TIEREN
PHAGE ANTIBACTÉRIEN, COMPOSITION DE TRAITEMENT, DÉSINFECTANT, ALIMENT, KIT D'IDENTIFICATION DE BACTÉRIES, PROCÉDÉ DE PRODUCTION D'UNE COMPOSITION DE TRAITEMENT, PROCÉDÉ D'ÉLIMINATION DE BACTÉRIES, PROCÉDÉ D'IDENTIFICATION DE BACTÉRIES ET PROCÉDÉ DE TRAITEMENT D'ANIMAUX

(30) Priority: 22.05.2018 JP 2018097751
(43) Date of publication of application: 31.03.2021
(73) Proprietor: Jichi Medical University, Tokyo 102-0093 (JP)
(72) Inventor: CUI, Longzhu, Shimotsuke-shi, Tochigi 329-0498 (JP); KIGA, Kotaro, Shimotsuke-shi, Tochigi 329-0498 (JP)
(74) Representative: Grünecker Patent- und Rechtsanwälte PartG mbB
(86) International application number: PCT/JP2019/016801
(87) International publication number: WO 2019/225246

(56) References cited:
- WO-A1-2014/124226
- WO-A1-2016/205276
- WO-A1-2017/173229
- WO-A2-2015/034872
- WO-A2-2017/066588
- WO-A2-2017/139505
- JP-A- 2017 513 489
- JP-A- 2017 537 643
- KIGA K ET AL: "Development of CRISPR-Cas13a-based antimicrobials capable of sequence-specific killing of target bacteria", NATURE COMMUNICATIONS, vol. 11, no. 1, 1 December 2020 (2020-12-01), XP055888259, Retrieved from the Internet <URL:https://www.nature.com/articles/s41467-020-16731-6.pdf> DOI: 10.1038/s41467-020-16731-6
- MAHAS, A. ET AL.: "Harnessing CRISPR/Cas systems for programmable transcriptional and post-transcriptional regulation", BIOTECHNOLOGY ADVANCES, vol. 36, no. 1, 29 November 2017 (2017-11-29), pages 295 - 310, XP055656240
- BIKARD, D. ET AL.: "Exploiting CRISPR-Cas nucleases to produce sequence-specific antimicrobials", NAT. BIOTECHNOL., vol. 32, no. 11, 2014, pages 1146 - 1150, XP055545750, DOI: 10.1038/nbt.3043
- CITORIK, R. J. ET AL.: "Sequence-specific antimicrobials using efficiently delivered RNA-guided nucleases", NAT. BIOTECHNOL., vol. 32, no. 11, 2014, pages 1141 - 1145, XP055545744, DOI: 10.1038/nbt.3011
- ABUDAYYEH, 0. 0. ET AL.: "C2c2 is a single-component programmable RNA-guided RNA-targeting CRISPR effector", SCIENCE, vol. 353, no. 6299, 2016, XP055407082, DOI: 10.1126/science.aaf5573
- PARK, J. Y . ET AL.: "Genetic engineering of a temperate phage-based delivery system for CRISPR/Cas9 antimicrobials against Staphylococcus aureus", SCIENTIFIC REPORTS, vol. 7, 2017, XP055550179, DOI: 10.1038/srep44929
- BIKARD, D. ET AL.: "Using CRISPR-Cas systems as antimicrobials", CURR. OPIN. MICROBIOL., vol. 37, 2017, pages 155 - 160, XP085276928, DOI: 10.1016/j.mib.2017.08.005

## Description

### Technical field

The present invention particularly relates to an antibacterial phage, a therapeutic composition, a bactericidal agent, a bacteria identification kit, therapeutic composition manufacturing method, an ex vivo bacteria elimination method, an ex vivo bacteria identification method, and uses of the antibacterial phage in a bacteria elimination or identification method and an animal therapeutic method.

### Background Art

In recent years, a technique called genome editing that modifies a target DNA by using a site-specific nuclease has attracted attention. For example, Patent Document 1 describes a technique for genome editing by using CRISPR (Clustered Regularly Interspaced Short Palindromic Repeats) / Cas9(Crispr associated protein 9). In CRISPR / Cas9, a guide RNA including a sequence complementary to the target site of DNA and Cas9 nuclease are injected into the cell. Then, the guide RNA specifically binds to the target site. Then, the Cas9 protein binds and cleaves the DNA so as to cover the guide RNA and the DNA.

On the other hand, in recent years, the emergence of drug resistant bacteria that antibacterial drugs do not work and their rapid spread have become problems. In other words, since drug resistant bacteria appear at a speed far faster than the development of antibacterial drugs, bacterial infections have become a global problem that threatens human health again.

Here, conventionally, phage therapy, which is an antibacterial treatment method by using a bacteriophage (hereinafter just referred to as "phage"), has been known. The phage therapy is a therapeutic method that uses the lytic activity of a phage, which is a virus that infects bacteria, to kill them. It was attempted with the discovery of phages in 1915 and is still being applied clinically in some areas of Eastern Europe. The conventional phage have a lower bactericidal efficacy than antibacterial agents, and therefore, it has limitations as therapeutic products for bacterial infections.

### Citation List

### Patent Literature

[Patent Document 1] PCT Publication No. 2014/204726
[Patent Document 2] WO 2014/124226 discloses compositions and methods for selectively reducing the amount of antibiotic resistant and/or virulent *S.aureus* bacteria in a mixed bacteria population. A phagemid comprising a CRISPR-Cas9 system targeting, e.g. an antibiotic resistance is disclosed.
[Patent Document 3] WO 2015/034872 describes a method of delivering to microbial cells a bacteriophage with at least one nucleic acid encoding a programmable Cas9 nuclease and a gRNA and/or tacrRNA targeting a bacterial antibiotic or toxin gene.

### Non-patent Literature

[Non-Patent Document 1] Shmakov, S, et al., "Discovery and Functional Characterization of Diverse Class 2 CRISPR-Cas Systems", Mol. Cell, 2015, Nov 5, 60(3), p. 385-397
[Non-Patent Document 2] Abudayyeh, OO, et al., "C2c2 is a single-component programmable RNA-guided RNA-targeting CRISPR effector.", Science, 2016, Aug 5, 353(6299), aaf5573
[Non-Patent Document 3] Gootenberg, JS, et al., "Nucleic acid detection with CRISPR-Cas13a/C2c2", Science, 2017, Apr 28, 356(6336), p. 438-442

### Summary of the Invention

### (Technical Problem)

However, even if conventional genome editing is applied to phages and used for the treatment of resistant bacteria, CRISPR / Cas9 only cleaves the genomic DNA of the bacteria, so that the bactericidal effect cannot be obtained by the gene repair mechanism of the bacteria themselves. In some cases, it could induce unexpected genetic evolution of the bacteria. Furthermore, the drug resistance gene in the bacteria is often on a plasmid, and in such a case, CRISPR / Cas9 for targeting a genomic DNA does not have any bactericidal effect.

Therefore, more effective phage has been required for the treatment of drug-resistant bacteria, and the like.

The present invention has been made in view of such a situation, and an object of the present invention is to solve the above-mentioned problems.

### (Solution to Problem)

An antibacterial phage according to the present invention includes CRISPR-Cas13a having a target sequence that recognizes a specific gene as a target,
wherein the target sequence is designed as a spacer sequence for crRNA of 14 to 28 bases,
wherein the specific gene is a drug resistance gene or a pathogenic gene included in the genome and / or plasmid of a bacterium of one or any combination of:
   Methicillin-Resistant Staphylococcus aureus (MRSA), Vancomycin-Resistant Staphylococcus aureus(VRSA), Vancomycin-Resistant enterococci (VRE), Penicillin-Resistant Streptococcus Pneumoniae (PRSP), Multidrug-resistant Pseudomonas aeruginosa (MDRP), Multiple Drug-Resistant Acinetobacter (MDRA), carbapenem-resistant Pseudomonas aeruginosa (CRPA), carbapenem-resistant Serratia (CRSA), third-generation cephalosporin-resistant pneumoniae (3GCRKP), third-generation cephalosporin-resistant E. coli (3GCREC), fluoroquinolone-resistant E. coli (FQREC), colistin-resistant E. coli (ColR-EC), or wherein the specific gene is a toxin gene or a pathogenic gene included in the genome and / or a plasmid of a bacterium of one or any combination of: Staphylococcus aureus, Clostridium botulinum, Vibrio cholerae, Escherichia coli, Vibrio parahaemolyticus, Clostridium tetani, Clostridium perfringens, Streptococcus pyogenes, Clostridium difficile, Bordetella pertussis, Corynebacterium diphtheriae, Shigella dysenteriae, Bacillus anthracis, Pseudomonas aeruginosa, Listeria monocytogenes, and Streptococcus pneumoniae.

The antibacterial phage according to the present invention is, wherein the drug resistance gene or pathogenic gene is one or any combination of: mecA, vanA, vanB, vanC, vanD, vanE, vanG, vanL, vanM, vanN, pbp1a, pbp2b, pbp2x, bla_{IMP}, bla_{VIM}, bla_{OXA}, bla_{SHV}, bla_{SME}, bla_{NDM}, bld_{IMI} bla_{TEM}, bla_{CMY}, bla_{GES}, bla_{CTX-M}, bla_{KPC}, aac(6'), parC, gyrA, qnrA, mcr, and mutation thereof.

The antibacterial phage according to the present invention is, wherein: the toxin gene includes one or any combination of: enterotoxin, botulinum neurotoxin, cholera toxin, heat-resistant enterotoxin, heat-resistant enterotoxin, heat-labile enterotoxin, Shiga toxin, tetanus toxin, alpha toxin, leucocidin, beta toxin, toxic shock syndrome toxin, streptidine O, erythrogenic toxin, alpha hemolytic toxins, cytotoxic necrotizing factors I, toxin A, toxin B, pertussis toxin, diphtheria toxin, adhesin, secretion (permeation) apparatus, lysates, and gene for producing superantigens.

The antibacterial phage according to the present invention is, wherein the target sequence is a specific 14 - 28 base sequence of the target gene sequence.

The antibacterial phage according to the present invention is, wherein the target sequence includes: SEQ ID NO: 7, SEQ ID NO: 17, SEQ ID NO: 22, SEQ ID NO: 26, SEQ ID NO: 27, SEQ ID NO: 28, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52.

The antibacterial phage according to the present invention is, wherein the specific gene causes drug resistance by nucleic acid acquisition and / or nucleic acid mutation.

The antibacterial phage according to the present invention is, wherein the target is a plurality of sequences, and each of a plurality of specific genes is recognized as the target.

A therapeutic composition according to the present invention including the antibacterial phage.

A bactericidal agent according to the present invention includes the antibacterial phage.

A bacteria identification kit according to the present invention includes the antibacterial phage.

A therapeutic composition manufacturing method according to the present invention including the steps of: transforming and/or infecting a phagemid having CRISPR, Cas13a, and a packaging sequence with a target sequence that recognizes a specific gene as a target, and helper phage with a phage-synthesizing bacteria, and lysing and / or releasing out of the cell; and obtaining constructed antibacterial phage.

An antibacterial phage including CRISPR-Cas13a for use in a bacteria elimination method according to the present invention including the steps of: applying antibacterial phage including CRISPR-Cas13a having a target sequence that recognizes a specific gene as a target; and eliminating bacteria having the specific gene.

The antibacterial phage including CRISPR-Cas13a for use in a bacteria elimination method according to the present invention is, wherein the bacteria are present in bacterial flora of humans or animals.

The ex vivo bacteria elimination method according to the present invention is, wherein the bacteria are present in food, and / or an environment.

An antibacterial phage including CRISPR-Cas13a for use in a bacteria identification method according to the present invention includes the steps of: infecting bacteria with antibacterial phage including CRISPR-Cas13a having a target sequence that recognizes a specific gene as a target; and determining and / or testing whether the bacteria have the specific gene.

In one aspect of the bacteria identification method according to the present invention, the antibacterial phage includes a sequence of an antibiotic resistance gene.

In another aspect of the bacteria identification method according to the present invention the bacteria are bacteria being present in the bacterial flora in humans, animals, foods, or the environment, and / or genetically modified bacteria.

An antibacterial phage including CRISPR-Cas13a for use in an animal therapeutic method according to the present invention includes: applying antibacterial phage including CRISPR-Cas13a having a target sequence that recognizes a specific gene as a target; treating infectious diseases caused by bacteria having the specific gene in the animal other than a human.

### (Effect of the Invention)

According to the present invention, as configured to include CRISPR-Cas13a having a target sequence that recognizes a specific gene as a target, an antibacterial phage capable of effectively treating drug-resistant bacteria, toxin-producing bacteria, or the like, can be achieved.

### Brief Description of the Drawings

FIG. 1 is a conceptual diagram of an antibacterial treatment method by using antibacterial phage according to the first embodiment of the present invention;
FIG. 2A is a diagram and a photograph showing a bactericidal effect of a bacterium having a specific gene according to Example 1 of the present invention by CRISPR-Cas13a;
FIG. 2B is a diagram and a photograph showing a bactericidal effect of a bacterium having a specific gene according to Example 1 of the present invention by CRISPR-Cas13a;
FIG. 2C is a diagram and a photograph showing a bactericidal effect of a bacterium having a specific gene according to Example 1 of the present invention by CRISPR-Cas13a;
FIG. 3 is a conceptual diagram of synthesis and experiment of the antibacterial phage according to Example 1 of the present invention;
FIG. 4 is a photograph showing a bactericidal effect of bacteria by the antibacterial phage infection according to Example 1 of the present invention;
FIG. 5 is a diagram and a photograph showing growth suppression of specific gene-carrying E. coli by the antibacterial phage infection according to Example 1 of the present invention;
FIG. 6 is a photograph showing the bactericidal activity of each target sequence according to Example 1 of the present invention;
FIG. 7 is a diagram showing the amount of information by the web logo of the target sequence in which strong bactericidal activity according to Example 1 of the present invention was confirmed;
FIG. 8 is a graph counting the number of colonies in the photograph shown in FIG. 6;
FIG. 9 is a photograph showing a determination result of a carbapenem resistance gene according to Example 1 of the present invention;
FIG. 10 is a photograph showing a determination result of a colistin resistance gene according to Example 1 of the present invention;
FIG. 11 is a diagram showing the results of bacterial flora modification by antibacterial phage according to Example 2 of the present invention;
FIG. 12 is a conceptual diagram of a method for investigating the optimum CRISPR sequence in the bactericidal effect of antibacterial phage according to Example 2 of the present invention;
FIG. 13 is a graph showing a bactericidal effect of antibacterial phage according to Example 2 of the present invention;
FIG. 14 is a diagram and a photograph showing the result of increasing the sensitivity of detection of a specific gene according to Example 2 of the present invention;
FIG. 15 is a photograph showing the results of identifying carbapenem resistance genes by using the method shown in FIG. 14;
FIG. 16 is a photograph showing the results of identification between the Shiga toxin-producing gene and the carbapenem resistance gene according to Example 2 of the present invention;
FIG. 17 is a diagram and a photograph showing the results of identification of carbapenem resistance genes of the clinically isolated E. coli strain according to Example 2 of the present invention;
FIG. 18 is a graph showing the therapeutic effect of antibacterial phage on moth larvae infected with the clinically isolated Escherichia coli strain according to Example 2 of the present invention; and
FIG. 19 is a graph showing the results of selective killing of bacteria having the resistance gene mecA of the methicillin-resistant Staphylococcus aureus strain according to Example 2 of the present invention and identification of mecA-positive bacteria.

### Description of the Embodiments

### <First Embodiment>

Considering the history of antibacterial drug development and the transition of resistant bacteria, the emergence of resistant strains is inevitable, and further, it is feared that there is no evolutionary adaptive drug that can respond to the emergence of new resistant strains. However, CRISPR / Cas9 as described in Patent Document 1 could not be applied to antibacterial treatment.

Therefore, the present inventors sought by using the CRISPR-Cas13a, which is a CRISPR system targeting RNA, described in Non-Patent Document 1. As described in Non-Patent Document 2, Cas13a was discovered in 2015, and its function was elucidated the following year. Although the CRISPR-Cas13a targets the RNA, Cas13a enzyme recognizes once a target RNA in a sequence-specific manner, and it rapidly changes the enzyme to degrade the various RNA sequence-nonspecifically. Therefore, Non-Patent Document 3 shows a method for detecting RNA degraded by Cas13a in order to detect DNA of an infectious pathogen. This method is just a detection method that is performed after extracting nucleic acid from bacteria, and it is completely unrelated to antibacterial treatment.

Actually, Cas13a of Non-Patent Document 2 uses a method of transforming a plasmid into bacteria, and therefore it cannot be applied to antibacterial treatment.

On the other hand, the present inventors considered the possibility of introducing the CRISPR-Cas13a into bacterial cells and using it as a bactericidal agent. Specifically, the present inventor has a conception of producing antibacterial phage, which is an artificially designed and manufactured synthetic phage, by designing CRISPR-Casl3a having a target sequence (target gene recognition sequence) that recognizes a resistance gene sequence, or the like, and by including (loading) the DNA encoding this in the phage, and the present invention is completed by repeating diligent experiments.

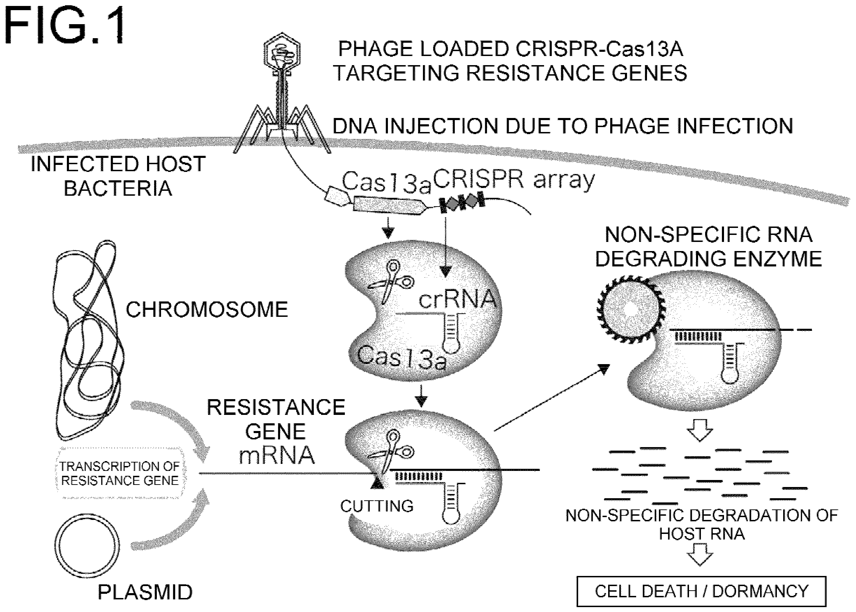

FIG. 1 shows the concept of an antibacterial treatment method by using antibacterial phage containing CRISPR-Cas13a defined in the claims.

The antibacterial phage as defined in the claims utilizes the specific nucleotide sequence recognition of CRISPR and the non-specific nucleotide sequence RNA degradation activity of Cas13a. When the antibacterial phage e-f as defined in the claims is infected with a target bacterium, Cas13a synthesized in the bacterium recognizes and cleaves the resistance gene mRNA derived from a chromosome or a plasmid. Cas13a incorporating the target sequence is transformed into a non-specific RNA-degrading enzyme, which induces cell death or dormancy associated with degradation of the host bacterial RNA. That is, by loading CRISPR-Cas13a DNA having a DNA sequence of a target sequence of about 20 bases on an antibacterial phage, antibacterial treatment that selectively kills only a specific bacterium having a specific gene becomes possible.

Hereinafter, an antibacterial phage, a therapeutic composition, a bactericidal agent, a bacterial identification kit, a therapeutic composition manufacturing method, and uses antibacterial phage in a bacteria elimination method, a bacteria identification method, and an animal therapeutic method as defined in the claims are specifically described in detail.

The antibacterial phage according to the first embodiment of the present invention is characterized in that including CRISPR-Cas13a having a target sequence that recognizes a specific gene as a target.

Here, the specific gene of the present embodiment is an arbitrary gene that makes the bacterium distinguishable into a specific genotype. The specific genotype may be, for example, even in the same type of bacterium, sufficient to be capable for identifying by the gene sequence of genomic DNA, plasmid in the bacterium, other gene not in the genome, or the like, and the phenotype does not necessarily have to be different. Further, the specific gene of the present embodiment may be a bacterium gene itself, a recombinant gene, a gene introduced into a genome, a plasmid, or the like. Furthermore, the specific gene does not necessarily have to be translated into a protein.

More specifically, the specific gene of the present embodiment may be, for example, a drug resistance gene of a drug-resistant bacterium, a various toxin gene, a gene encoding proteins associated with being weak virulent or strong virulent, an enzyme gene for producing a specific metabolite, a gene for identifying the bacterium, a reporter gene used for transformation, a sequences having restriction enzymes and sticky ends used for genetic modification, a repeat sequence, a "gene" indicating another genotype in a broad sense.

That is, the antibacterial phage of the present embodiment can target a bacterium having any specific gene, such as a bacterium having a specific virulence factor, or the like, by changing the design target sequence.

As a specific example, when this specific gene is a drug resistance gene, it may be included in the genome and / or a plasmid of a bacterium having one or any combination of the group including: a various pathogenic E. coli strain (Escherichia coli), methicillin-resistant Staphylococcus aureus (MRSA), vancomycin-resistant Staphylococcus aureus (VRSA), vancomycin-resistant enterococci (VRE), penicillin-resistant Streptococcus pneumoniae (PRSP), multidrug-resistant Pseudomonas aeruginosa (MDRP), multidrug-resistant Acinetobacter (MDRA), multidrug-Resistant Acinetobacter, carbapenem-resistant Pseudomonas aeruginosa (CRPA), carbapenem-resistant Serratia (CRSA), third generation cephalosporin-resistant Klebsiella pneumoniae (3GCRKP), 3rd generation cephalosporin-resistant E. coli (3GCREC), fluoroquinolone-resistant E. coli (FQREC), colistin resistant E. coli (ColR-EC).

In this case, these drug resistance genes includes any one or any combination of the group: mecA, vanA, vanB, vanC, vanD, vanE, vanG, vanL, vanM, vanN, pbp1a, pbp2b, pbp2x, blaIMP, bla_{VIM}, bla_{OXA}, bla_{SHV}, bla_{SME}, bla_{NDM}, bla_{IMI,} bla_{TEM,} bla_{CMY}, bla_{GES}, bla_{CTX-M}, bla_{KPC}, aac(6'), parC, gyrA, qnrA, mcr, and a type of mutation thereof.

Examples of these drug-resistant bacteria (types of resistant bacteria), drug-resistant genes (gene names encoding target RNA), and phages for antibacterial phage used in this embodiment (bacteriophage) are summarized and shown in Table 1 below:

**[Table 1]**

| No. | TYPES OF RESISTANT BACTERIA | GENE NAME ENCODING TARGET RNA | TYPES OF BACTERIOPHAGE (ABOUT 2 - 3 TYPES) |
|---|---|---|---|
| 1 | METHICILLIN-RESISTANT STAPHYLOCOCCUS AUREUS (MRSA) | *mecA* | NM1, SaPIbov1, SaPIbov2, SaPI1, K, S25-1, S24-3, 80α, OTHER LYSOGENIZED PHAGE |
| 2 | VANCOMYCIN-RESISTANT STAPHYLOCOCCUS AUREUS(VRSA) | *vanA, vanB* | NM1, SaPIbov1, SaPIbov2, SaPI1, K, S25-1, S24-3, 80α, OTHER LYSOGENIZED PHAGE |
| 3 | VANCOMYCIN-RESISTANT ENTEROCOCCI (VRE) | *vanA, vanB, vanC, vanD, vanE, vanG, vanL, vanM, vanN* | ϕEf11, V583, OTHER LYSOGENIZED PHAGE |
| 4 | PENICILLIN-RESISTANT STREPTOCOCCUS PNEUMONIAE (PRSP) | *pbp1a, pbp2b pbp2x* | Dp-1, HB-1, EJ-1, OTHER LYSOGENIZED PHAGE |
| 5 | MULTIDRUG-RESISTANT PSEUDOMONAS AERUGINOSA (MDRP) | *bla*_{IMP[NOTE 1]} *bla*_{VIM[NOTE2]}, *bla*_{OXA[NOTE6]}, *aac(6'), parC, gyrA* | gh-1, philBB-PF7A, phi15, KPP12, OTHER LYSOGENIZED PHAGE |
| 6 | MULTIPLE DRUG-RESISTANT ACINETOBACTER (MDRA) | *bla*_{IMP[NOTE]} *bla*_{SHV[NOTE8]}. *bla*_{SME[NOTE 10]} *aac(6'), gyrA, parC* | IsxxfAB78, F1245/05, OTHER LYSOGENIZED PHAGE |
| 7 | CARBAPENEM-RESISTANT PSEUDOMONAS AERUGINOSA (CRPA) | *bla*_{IMP[NOTE1]}, *bla*_{VIM[NOTE 2]}, *bla*_{NDM[NOTE 3]}, *bla*_{IMI[NOTE 7]}, | gh-1, philBB-PF7A, phi15, OTHER LYSOGENIZED PHAGE |
| 8 | CARBAPENEM-RESISTANT SERRATIA (CRSA) | *bla*_{*T*EM[NOTE 9]}, *bla*_{CMY[NOTE 12]}, *bla*_{GES[NOTE5]} | phiMAM1, CBH8, OTHER LYSOGENIZED PHAGE |
| 9 | THIRD-GENERATION CEPHALOSPORIN-RESISTANT PNEUMONIAE (3GCRKP) | *bla*_{CTX-M[NOTE11]}, *bla*_{TEM[NOTE 9]}, *bla*_{KPC[NOTE4]}, *bla*_{SHV[NOTE8]} | K11, KP32, vB_Klox-2, OTHER LYSOGENIZED PHAGE |
| 10 | THIRD-GENERATION CEPHALOSPORIN-RESISTANT E. COLI (3GCREC) | *bla*_{IMP[NOTE1]} | M13, T6, 80, λ, OTHER LYSOGENIZED PHAGE |
| 11 | FLUOROQUINOLONE-RESISTANT E. COLI (FQREC) | *gyrA, parC, qnrA* | M13, T6, 80, λ, OTHER LYSOGENIZED PHAGE |
| 12 | COLISTIN-RESISTANT E. COLI (COLR-EC) | *mcr* _{[NOTE 13]} | M13, T6, 80, λ, OTHER LYSOGENIZED PHAGE |

| | | | |
|---|---|---|---|
| NOTE 1. *bla*_{IMP}: *bla*_{IMP-1}, *bla*_{IMP-6}, *bla*_{IMP-7}, *bla*_{IMP-10}, *bla*_{IMP-11}, *bla*_{IMP-15}, bla_{IMP-19}, *bla*_{IMP-31}, *bla*_{IMP-35}, *bla*_{IMP-37}. NOTE 2. *bla*_{VIM}: *bla*_{VIM-1}, *bla*_{VIM-2,} *bla*_{VIM-4}*, bla*_{VIM-5}, *bla*_{VIM-7}*, bla*_{VIM-10}, *bla*_{VIM-18}, *bla*_{VIM-19}*, bla*_{VIM-25}. NOTE 3. *bla*_{NDM}: *bla*_{NDM-1}, b*la*_{NDM-2}, *bla*_{NDM-3}, *bla*_{NDM*-*4}, *bla*_{NDM-5}, *bla*_{NDM-6}, *bla*_{NDM-7}, *bla*_{NDM***-*** 8,} *bla*_{NDM-9}, *bla*_{NDM-10}. NOTE 4, *bla*_{KPC}: *bla*_{KPC-1}, *bla*_{KPC-2}*, bla*_{KPC-3}*, bla*_{KPC-5}*, bla*_{KPC-7}*,* bla_{KPC-10}, *bla*_{KPC-11}, *bla*_{KPC-14}*, bla*_{KPC-15}. NOTE 5. *bla*_{GES}: *bla*_{GES-2}*_{,} bla*_{GES-4}, *bla*_{GES-5}, *bla*_{GES-18}. NOTE 6. *bla*_{OXA}: *bla*_{OXA-11}, *bla*_{OXA-15}, *bla*_{OxA-23}, *bla*_{OXA-24}*, bla*_{OXA-18}, *bla*_{OxA-58}, *bla*_{OXA-146}, *bla*_{OXA-162}, *bla*_{OXA-181}. NOTE 7. *bla*_{IMI}: *bla*_{IMI-1}, *bl*a_{IMI-2}, *bla*_{IMI-3}. NOTE 8. *bla*_{SHV}: *bla*_{SHV-1}, *bla*_{SHV-10}. NOTE 9. *bla*_{TEM}: *bla*_{TEM-1}, *bla*_{TEM-10}, *bla*_{TEM-30}. NOTE 10. *bla*_{SME}: *bla*_{SME-1}, *bla*_{SME-2}, *bla*_{SME-3}. NOTE 11. *bla*_{CTX-M}: *bla*_{CTX-M-1}, *bla*_{CTX-M-2}, *bla*_{CTX-M-9}, *bla*_{CTX-M-19.} NOTE 12. *bla*_{CMY}: *bla*_{CMY-1}, *bla*_{*CMY*-2}*.* NOTE 13. *mcr*: *mcr*₋₁*, mcr-₂., mcr-*₃*.* | | | |

As shown in this table, drug resistance genes include their respective mutants (variations).

In addition, these drug resistance genes may be horizontally transmitted to other kind of bacteria, which the classification as bacteria may be different.

In addition, the specific gene may be one that causes drug resistance by nucleic acid acquisition and / or nucleic acid mutation.

Specifically, since resistant bacteria may be originated by acquisition of foreign DNA or RNA (nucleic acid) and / or nucleic acid mutation, the nucleic acid mutation may be a specific gene targeted by the antibacterial phage according the present embodiment. In this case, the antibacterial phage of the present embodiment can counter the bacteria having the nucleic acid mutation that has caused drug resistance as the "specific gene" by modifying the target sequence in response to this mutation.

Further, the target sequence of the antibacterial phage according to the first embodiment of the present invention is characterized by being a base sequence designed to recognize the specific gene as a target. On this basis, when the target sequence of the present embodiment is designed as a spacer sequence of crRNA of 14 to 28 bases, it is preferable that the target at the 10th base in the DNA base sequence when packaged in the antibacterial phage is T. Specifically, the target sequence may be, for example, SEQ ID NO: 7, SEQ ID NO: 17, SEQ ID NO: 22, SEQ ID NO: 26, SEQ ID NO: 27, SEQ ID NO: 28, and SEQ ID NO: 36.

Here, as shown in the following example, in the antibacterial phage having CRISPR-Cas13a of the present embodiment, even if a location at a random position within the specific gene is simply selected as the crRNA sequence, there is a difference in the bactericidal effect. For this reason, as a result of diligent experiments conducted by the present inventors, we have found that it is possible to optimize the efficiency of nonspecifically degrading the RNA of Cas13a to cause the bacterium to be cell-death by using a sequence in which the 10th base is T (thymine) on the 5'to 3'side as a crRNA spacer sequence of 14 to 28 bases in the DNA base sequences in the specific gene. As an example of such a sequence, each nucleic acid sequence of SEQ ID NO: 7, SEQ ID NO: 17, SEQ ID NO: 22, SEQ ID NO: 26, SEQ ID NO: 27, SEQ ID NO: 28, and SEQ ID NO: 36 in the following sequence listing is shown. Furthermore, as a specific example, the base sequence of crDNA loaded in the antibacterial phage may be designed as a complementary sequence as shown in the following examples. In addition, when crDNA targets a transcribed RNA, the target in the RNA is U (Uracil).

Furthermore, as shown in the second embodiment as described later, sequences other than these may be accepted as target sequences of antibacterial phage.

In addition, there are a plurality of target sequences, and a plurality of specific genes may be configured to be recognized as targets. That is, the antibacterial phage of the present embodiment can have a plurality of target sequences in one phage. At this time, since it is possible to include about 1 to a dozen spacer sequences of CRISPR, a plurality of target sequences may be included within the range that can be included in the capsid of the phage. The plurality of target sequences may be arranged consecutively or as a plurality of sites in the sequence of crRNA, or it may be configured to have a plurality of crRNAs having different target sequences in the phage. This makes it possible to apply one antibacterial phage to a plurality of resistant bacteria. Furthermore, in the case of multidrug-resistant bacteria having a plurality of resistance genes, it is possible to improve the antibacterial efficiency by CRISPR-cas13a.

Further, the antibacterial phage according to the first embodiment of the present invention can also be used for the use of a therapeutic composition including the antibacterial phage. That is, by administering the antibacterial phage of the present embodiment for treatment, it can be used in an antibacterial treatment method for selectively killing a specific genotype bacterium. Specifically, CRISPR-Cas13a DNA that specifically recognizes the target RNA is synthesized and packaged in antibacterial phage (included in the capsid) to selectively kill the target (having a specific gene) bacterium, and an antibacterial treatment method can be realized. This makes it possible to enable antibacterial treatment of infectious diseases that are difficult to treat with antibacterial agents, including infectious diseases of drug-resistant bacteria.

In this therapeutic composition manufacturing method, the steps of: transforming and/or infecting a phagemid having CRISPR, Cas13a, and a packaging sequence with the target sequence that recognizes a specific gene as a target, and helper phage with a phage-synthesizing bacteria, and lysing and / or releasing out of the cell, and obtaining constructed antibacterial phage are included.

Specifically, in the therapeutic composition of the present embodiment, it is possible to use synthesized CRISPR-Cas13a DNA that specifically recognizes the target RNA, which is loaded into antibacterial phage (packaging, included in capsid), and various formulations similar to the conventional antibacterial treatment method by using antibacterial phage (antibacterial phage therapy). As the phage synthesizing bacterium, for example, a general bacterium such as E. coli, or the like, can be used.

In addition, the therapeutic composition according to the first embodiment of the present invention can be administered together with any pharmaceutically acceptable carrier composition. Examples of the carrier composition include isotonic solutions containing physiological saline, glucose and other adjuvants, such as D-sorbitol, D-mannose, D-mannitol, sodium chloride and the like, suitable solubilizers such as alcohols, specifically ethanol, poly-alcohols such as propylene glycol, polyethylene glycol, nonionic surfactants such as polysorbate 80 (TM), HCO-50, and the like, can be mentioned, and, however, it is not limited to them. In addition, suitable excipients, and the like, may be further included.

Also, the therapeutic compositions of this embodiment may include suitable pharmaceutically acceptable carriers for preparing a pharmaceutically acceptable carrier composition. The carrier may include biocompatible materials such as silicone, collagen, gelatin, or the like. Alternatively, it may be various emulsion liquid. Further, for example, one or more pharmaceutical additives selected from diluents, fragrances, preservatives, excipients, disintegrants, lubricants, binders, emulsifiers, plasticizers, and the like may suitably be included.

The therapeutic composition according to the first embodiment of the present invention may be formulated in a dosage form suitable for administration for oral administration, by using a pharmaceutically acceptable carrier well known in the art.

The way of administration of the pharmaceutical composition according to the present invention is not particularly limited, and oral administration or non-oral administration is possible. The non-oral administration can be, for example, intravenous, intraarterial, subcutaneous, intradermal, intramuscular, intraperitoneal administration, or direct administration to the bacterial flora or infected site. The administration method may be carried out in the same manner as typical phage therapy.

Further, in order to use the therapeutic composition according to the first embodiment of the present invention for the above-mentioned treatment, the administration interval and the dose can appropriately be selected and changed according to various conditions such as the condition of the disease and the condition of the subject.

The single dose and the number of administrations of the therapeutic composition according to the first embodiment of the present invention can be selected and changed as appropriate depend on the purpose of administration and further depending on various conditions such as the age and weight of the patient, symptoms and severity of disease.

The number and duration of administration may be only once, or may be administered once to several times a day for several weeks, the state of the disease may be monitored, and the administration may be performed again or performed repeatedly depending on the state.

The composition of the present invention can also be used in combination with other compositions, and the like. Further, the composition of the present invention may be administered at the same time as other compositions, or may be administered at intervals, but the order of administration is not particularly limited. Further, in the first embodiment of the present invention, the period during that the disease is improved or alleviated is not particularly limited, but may be temporary improvement or alleviation, or may be improvement or alleviation for a certain period.

In addition, the therapeutic composition of the first embodiment of the present invention can be the subject of treatment for an organism, a part of the body of the organism, or a part thereof extracted or excreted from the organism. This organism is an animal other than a human including, for example, livestock animal species, wild animals, or the like. Therefore, the therapeutic composition according to the first embodiment of the present invention can also be used for animal treatment for treating animals. That is, the antibacterial phage of the present embodiment can also be used in an animal treatment method for various animals other than humans.

Specifically, in the animal therapeutic method, it is possible to treat infectious diseases caused by bacteria having the specific gene in animals other than humans by using antibacterial phage including CRISPR-Cas13a having a target sequence that recognizes the specific gene as the target.

The animal is also not particularly limited, and it includes a wide range of vertebrates and invertebrates. The vertebrates include fish, amphibians, reptiles, birds, and mammals. Specifically, for example, the mammals may include rodents, such as mice, rats, hamsters, guinea pigs, or rabbits, ferrets, dogs, cats, sheep, pigs, cows, horses, or non-human transgenic primates. In addition to the mammals, wild animals include fish, birds including poultry, reptiles, or the like. They also include a wide range of crustaceans including shrimp and insects, and other invertebrates such as squid. That is, the therapeutic composition according to the first embodiment of the present invention can be used not only for human treatment but also for animal treatment, livestock growth promotion, and the like.

Further, the bacteria elimination method is characterized in that includes the steps of: applying antibacterial phage including CRISPR-Cas13a having a target sequence that recognizes a specific gene as a target; and eliminating bacteria having the specific gene. Therefore, the composition including the antibacterial phage of the present embodiment can be used as the bactericidal agent (growth inhibitor). The bactericidal agent can also be included in an appropriate carrier, solution, or the like, as described above. It is also possible to add an auxiliary agent that eliminates bacterial peptidoglycan, biofilm, or the like. The bactericidal agent may be provided by being included in various antibacterial products such as hand wash solutions, mouthwashes, masks and napkins, air purifier filters, and the like.

By applying, spraying, or the like, the bactericidal agent according to the present embodiment, it is possible to eliminate specific genotype bacteria and suppress their growth from the focus of infection, bacterial flora, and environment. That is, it is possible to reduce bacteria having a specific gene present in the bacterial flora in humans, animals, and / or the environment.

In addition, in the bacteria elimination method defined in the claims, it is also possible to eliminate bacteria existing in food. This is because the antibacterial phage of the present embodiment does not infect humans. That is, the antibacterial phage of the present embodiment can selectively eliminate a specific genotype bacterium having a specific gene in food. Further, it is also possible to provide foods from which specific genotype bacteria have been eliminated in this way. This makes it possible to provide, for example, a safe food from which food poisoning bacteria that produce toxins have been reliably eliminated. Here, the antibacterial phage of the present embodiment can be used by being included in any of food cultivation, culture fishery, harvesting, or the like, including various vegetables, meat, seafood, processed foods, dairy products, and the like.

In addition, the bacteria identification method including the steps of: infecting bacteria with antibacterial phage including CRISPR-Cas13a having a target sequence that recognizes a specific gene as a target; and determining and / or testing whether the bacteria have the specific gene. Specifically, by designing CRISPR-Cas13a targeting the specific gene, loading it on the antibacterial phage of the present embodiment, and selectively killing the bacteria having the specific gene, the bacteria that has the specific gene and is the specific genotype can be easily detected. That is, the antibacterial phage of the present embodiment can also be used for identification and inspection.

Specifically, for example, by being the increase (growth) in the number of bacteria suppressed after simultaneously adding the target bacterium and the antibacterial phage of the present embodiment, or, by culturing on a plate, applying the antibacterial phage of the present embodiment, and confirming lysis, it is possible to easily determine (test) whether or not the specific gene is present in the bacterium. The specific gene that can be identified by this bacterial identification method may be the specific gene targeted by the above-mentioned bacterial elimination method.

As described above, the bacterium to be identified and / or tested by this bacteria identification method may be a bacteria present in the bacterial flora in humans, animals, foods, or the environment, and / or a genetically modified bacterium. It is also possible to provide a bacterial identification kit including the antibacterial phage according to the present embodiment.

As configured in this way, the following effects can be obtained.

Traditionally, antibacterial agents have played the most important role in the treatment of bacterial infections. However, due to the emergence of drug-resistant bacteria for which antibacterial drugs do not work and their rapid spread, existing antibacterial agents are being neutralized. For this reason, bacterial infections have become a global problem that threatens human health again. Specifically, the problem of resistant bacteria has become unsolvable due to the emergence of resistant bacteria at a speed far faster than the development of antibacterial drugs and the stalemate in the development of new antibacterial drugs.

On the other hand, the antibacterial phage according to the first embodiment of the present invention, by loading CRISPR-Cas13a on the antibacterial phage, it is possible to deal with resistant bacteria without using antibacterial agents. That is, since the specific gene targeted by CRISPR-Cas13a can be arbitrarily determined, bacteria of any specific genotype can be a sterilized target.

Further, since the antibacterial phage of the present embodiment targets the RNA transcribed by CRISPR-Cas13a, it is possible to effectively sterilize the target specific gene regardless of whether it is in a chromosome or a plasmid.

Further, since the antibacterial phage of the present embodiment targets RNA, evolutionary selective pressure is hard to apply, and resistant bacteria are less likely to be generated in principle. Furthermore, even for bacteria that are not sterilized due to different specific genotypes, antibacterial phage corresponding to the specific gene of such specific genotype may be designed and applied. Therefore, by appropriately selecting a specific gene to produce an antibacterial phage, it is possible to surely kill the specific genotype bacterium. That is, the antibacterial phage according to the first embodiment of the present invention can be an evolutionary adaptive drug that can respond by redesigning only the sequence recognition portion of CRISPR-Cas13a when a new resistant bacterium is generated. Also, it is also possible to modify the loaded antibacterial phage itself by using genetic engineering technology.

Further, in the antibacterial treatment method by using an antibacterial agent, there is a problem that the antibacterial range of the antibacterial agent, that is, the range of target bacteria exerting a bactericidal effect is wide, and the selectivity is low. For this reason, bacteria other than the target bacteria also have an antibacterial effect, and bacteria resistant to antibacterial agents have been selected, leading to induction of drug resistance, and the like. In addition, this also affected the balance of the host's normal flora by killing a wide range of bacteria.

On the other hand, in the antibacterial phage according to the first embodiment of the present invention, CRISPR-Cas13a can recognize a target sequence of about 20 bases, and this recognition requires almost 100% sequence homology. This selectivity enables highly selective antibacterial treatment that kills only the target bacteria. As a result, useful indigenous bacteria, and the like, are not sterilized, and the balance of the normal bacterial flora of the host can be maintained. In addition, by introducing CRISPR-Cas13a into infected bacterial cells via antibacterial phage limited by the bacterial species to be infected, the selectivity of the target bacterium can be further enhanced. That is, CRISPR-Cas13a recognizes and activates the target RNA sequence, enabling "tracking missile-type" antibacterial therapy that can selectively eliminate only specific genotype bacteria from the bacterial flora. Furthermore, it is possible to establish an antibacterial treatment method that can be applied to all bacterial species in humans, animals, and the environment.

Further, in the present embodiment, since the antibacterial phage, which is a synthetic phage that does not self-proliferate, is used, side effects due to mutation of the antibacterial phage itself can be suppressed. In addition, since the antibacterial phage does not self-proliferate, the possibility that the prophage is retained by the administered organism or bacteria in the environment is extremely low. Furthermore, in principle, resistant bacteria are less likely to occur. This enhances safety. Furthermore, even if the target gene is mutated, it can be dealt with by changing the probe (crRNA sequence) that recognizes the target RNA each time. In addition, since it is an antibacterial phage that does not self-proliferate, it is possible to accurately estimate the administration dose.

In addition, the antibacterial phage according to the first embodiment of the present invention can control gene expression in specific cells in a living body, and thus not only does it help clarify the physiological functions of cells, but it may also lead to the control of pathological conditions. This may lead to the development of new treatments.

Further, the antibacterial phage according to the first embodiment of the present invention only kills bacteria having a specific gene, and in addition, since it is derived from a natural substance, it is safe and has a low environmental load. Therefore, it is possible to provide safe and environmentally friendly bactericide and food.

In addition to treatment, the antibacterial phage according to the first embodiment of the present invention can also be applied to a bacterial identification method for detecting (identifying) a specific gene with high sensitivity. This makes it possible to reliably determine whether or not a specific resistance gene is present in, for example, a clinically isolated bacterium such as Escherichia coli, or the like. In such a case, it does not take time and effort because it is only visually observed that the bacteria do not grow or lyse, and since the amplification of nucleic acid is not required, it is not necessary to use a device such as PCR, so that it can become identified at low cost.

### <Second embodiment>

An example of producing an antibacterial phage, which is an artificially designed and manufactured synthetic phage, is described. In the example, a CRISPR-Casl3a having a target sequence (target gene recognition sequence) that recognizes a toxin gene as a specific gene is designed, and a DNA encoding this is included (loaded) in the phage.

As a specific example, when this specific gene is a toxin gene, it may be included in a bacterial genome and / or plasmid having one or any combination of groups including: Staphylococcus aureus, Clostridium botulinum, Vibrio cholerae, Escherichia coli, Vibrio parahaemolyticus, Clostridium tetani, Clostridium perfringens, Group A streptococcus (Streptococcus pyogenes), Clostridium difficile, Bordetella pertussis, Diphtheria (Corynebacterium diphtheriae), Shigella dysenteriae, Bacillus anthracis, Pseudomonas aeruginosa, Listeria monocytogenes, Streptococcus pneumoniae (Staphylococcus pneumoniae).

In this case, the toxin gene includes one kind or any combination of the group including: enterotoxin, botulinum neurotoxin, cholera toxin, heat-resistant enterotoxin, heat-labile enterotoxin, thermostable direct hemolysin, Shiga toxin, tetanus toxin, alpha toxin, leucocidin, beta toxin, toxic shock syndrome toxin, streptidine O, erythrogenic toxin, alpha hemolytic toxins, cytotoxic necrotizing factors I, toxin A, toxin B, pertussis toxin, diphtheria toxin, adhesin, secretion (permeation) apparatus, lysates, and gene for producing superantigens.

The types of these toxin-producing bacteria (types of bacteria), toxin genes (gene names encoding target RNA), and phages for antibacterial phage (bacteriophage) used in this embodiment are summarized in Table 2 below.

**[Table 2]**

| No. | BACTERIAL SPECIES | TOXIN |
|---|---|---|
| 1 | Staphylococcus aureus | enterotoxins, Toxic shock syndrome toxin (TSST-1), Exfoliatin toxin, alpha toxin, beta toxin, leukocidin |
| 2 | Clostridium botulinum | Botulinum toxin |
| 3 | Vibrio cholerae | Cholera enterotoxin (ctx) |
| 4 | Escherichia coli | E. coli LT toxin, E. coli ST toxin, hemolysin, Shiga toxin |
| 5 | Vibrio parahaemolyticus | thermostable direct hemolysin (TDH), TDH-related hemolysin (TRH) |
| 6 | Clostridium tetani | Tetanus toxin |
| 7 | Clostridium perfringens | Perfringens enterotoxin, perfingiolysin O |
| 8 | Streptococcus pyogenes | Erythrogenic toxin (streptococcal pyrogenic exotoxin SPE), streptolysin O |
| 9 | Clostridium difficile | toxin A, toxin B |
| 10 | Bordetella pertussis | Pertussis toxin (ptx), Adenylate cyclase toxin |
| 11 | Corynebacterium diphtheriae | Diphtheria toxin (dtx) |
| 12 | Shigella dysenteriae | Shiga toxin |
| 13 | Bacillus anthracis | Anthrax toxin (LF), Anthrax toxin (EF) |
| 14 | Pseudomonas aeruginosa | Exotoxin A |
| 15 | Listeria monocytogenes | listeriolysin |
| 16 | Streptococcus pneumoniae | pneumolysin |

As shown in this table, each of these toxin genes may have a genetic mutation (variation).

In addition, these toxin genes may be horizontally transmitted to other types of bacteria and may be classified differently as bacteria.

Further, the specific gene of the present embodiment may be a gene that causes a toxin by acquisition of nucleic acid.

Specifically, since the toxin-producing bacterium may be generated by the acquisition of foreign DNA or RNA (nucleic acid), this mutation by nucleic acid may be targeted to be the specific gene for the antibacterial phage.

In this case, the antibacterial phage can counteract the bacterium having the nucleic acid mutation that is produced the toxin as the "specific gene" by modifying the target sequence in response to this mutation.

The therapeutic composition, the treatment composition, the bactericide, the bacterial identification kit, the therapeutic composition manufacturing method, the use of the antibacterial phage in an bacterial elimination methods, a bacterial identification method, and animal therapeutic methods by using the antibacterial phage utilizing the toxin gene as the specific gene can be realized in the similar manner as in the first embodiment described above, and the similar effect can be obtained.

For example, in the use of the antibacterial phage in an animal therapeutic method as defined in the claims, it is possible to treat infectious diseases caused by bacteria carrying a specific gene in an animal other than a human by using antibacterial phage having CRISPR-Cas13a with the target sequence that recognizes the toxin gene as the target of the specific gene.

In addition, the antibacterial phage of the present embodiment may have a sequence of an antibiotic resistance gene. On this basis, it is also possible to determine and / or test whether or not the bacterium has the specific gene by using a medium including the antibiotic resistance gene.

Specifically, as shown in Example 2 described later, the antibacterial phage having the antibiotic resistance gene and is cultured on a plate containing and / or not containing this antibiotic, and this makes possible to determine and / or test whether the bacteria have the specific gene.

By incorporating an antibiotic resistance gene into an antibacterial phage, when cultured on a plate containing an antibiotic, the antibacterial phage is infected and only bacteria not having the specific gene grow. On the other hand, bacteria that have not been infected with the antibacterial phage cannot grow because of the antibiotics, and even if infected with antibacterial phage, bacteria having the specific gene is lysed. Here, the drug resistance gene, the toxin gene, or the pathogenic gene, which is detected as a specific gene, targets a gene other than the antibiotic resistance gene having in the antibacterial phage.

As configured in this way, it is possible to increase the detection sensitivity of a specific gene by antibacterial phage by about 100 times or more as compared with culturing in a medium containing no antibiotic.

In addition, in the above-mentioned first embodiment and the second embodiment, an example in which a drug resistance gene or a toxin gene is targeted as a specific gene has been described. Also, as for bacteria, an example of application to bacteria having such drug resistance gene is described.

However, the specific gene is an arbitrary gene, and any bacterium having the specific gene can be an antibacterial target.

That is, any gene that can be distinguished as the above-mentioned specific genotype can be targeted as the specific gene of the present embodiment. In addition, any bacterium having the specific gene can be targeted without specifying the bacterium. Specifically, as an example, any of Shigella (Shigella dysenteria) and enterohemorrhagic Escherichia coli (E. coli, EHEC) having the same specific gene of verotoxin can be targeted for antibacterial activity. In such a case, all the bacteria having a specific genotype can be made antibacterial by making the phage so that a plurality of bacteria are targeted at the same time.

Furthermore, the specific gene may include a gene that is expressed, regulated expression, or associated with a drug resistance gene or a toxin gene. In addition, the particular gene may have a pathogenic gene associated with the other pathogenicity.

In addition, the probe (crRNA sequence) that recognizes the target RNA of CRISPR-Cas13a according to the first embodiment and the second embodiment of the present invention can identify even a single base difference in the base sequence. Therefore, it can be applied to treatment of a cancer or a hereditary disease. In this case, it is possible to use not necessarily Cas13a for bacteria, but a similar enzyme that induces apoptosis, or the like, by non-specific RNA degradation action in cells other than eubacteria and leads to cell death.

Furthermore, the specific gene of the present embodiment may be a "gene" in a broad sense such as a sequence of a gene mutation targeted for cancer treatment or a hereditary disease, a single nucleotide substitution (SNP), or a repeat sequence. In this case, the bacterium itself can be administered therapeutically as a carrier of the drug. That is, it can also be applied to an application that a drug corresponding to the cell of the specific gene is contained in the cytoplasm of the target bacterium, or the like, and is dispersed around a tissue and target cells by lysis. In such case, it is also possible to provide the bacterium itself including the prophage. Further, it may also include prophage that is activated by a particular immune response.

Further, in the above-mentioned first embodiment and the second embodiment, an example in which a general bacteriophage is used as an antibacterial phage has been described. However, as the "antibacterial phage", it is also possible to use a virus, RNA virus, phagemid, or the like, which infects bacteria and is different from a general bacteriophage. In addition, the antibacterial phage may be provided in the state of prophage, which is a state integrated into the bacterial genome or plasmid. Moreover, for example, in the bacteria identification method in the present embodiment, phagemid can be introduced into cells by any method such as using electroporation and nanoparticles.

Further, in the bacterial identification method detection may be performed that does not necessarily require suppression of bacterial growth or complete lysis. In such case, for example, CAS13a binds to generate non-specific RNA-degrading activity, and RNA bound to various fluorescent probes is cleaved. Thereby, various reporters such as fluorescence may be induced to detect the presence or absence, the copy number, the expression level, or the like, about the specific gene. Even in this case, when detected by the bacterium identification method, the detected bacterium can be an antibacterial target. That is, the antibacterial phage can be used for antibacterial applications.

In addition, the antibacterial phage according to the first embodiment and the second embodiment of the present invention can be used in combination with other compositions, and the like. Furthermore, it is also possible to provide it as a "cocktail" including a plurality of types of antibacterial phages. Further, the composition of the present invention may be administered, sprayed, applied, or the like, at the same time as other compositions.

### [Example 1]

Hereinafter, the killing (bacteria elimination) and bacterial identification method by using antibacterial phage according to the first embodiment of the present invention is described more specifically as examples based on specific experiments. However, this embodiment is merely an example, and the present invention is not limited thereto.

### [Bactericidal effect of bacteria having a specific gene of CRISPR-Cas13a]

### (Preparation of tetracycline-induced bla_{IMp-1} expression vector)

The sequence of tetracycline-dependent expression control region is
amplified from pC008 (obtained from Dr. F. Zhang) with primers of TetReg SalI-f, "ATATGTCGACGCATGCTTAAGACCCACTTTC" and TetReg BamHI-r, "ATATGGATCCTTTCTCCTCTTTAGATCTTTTG." It was subcloned into the SalI-BamHI site of the pSP72 vector (hereinafter referred to as "pSP72 aTc-inducible vector").

Then, the sequence of bla_{IMP-1} (carbapenem resistance gene) was amplified from multidrug-resistant colorectal cocci by using primers of IMP-1 clo BamHI-f, "ATATGGATCC ATGAGCAAGTTATCTGTATTC" and IMP-1 clo EcoRI-r, "ATATGAATTCTTAGTTGCTTGGTTTTGATG", and it was cloned into the BamHI-EcoRI site of the pSP72 aTc-inducible vector ("pSP72 aTc-inducible IMP-1 vector", hereinafter referred to as "tetracycline-inducible bla_{Imp-1} expression vector").

### (Preparation of CRISPR-Cas13a expression vector targeting the bla_{IMP-1} gene)

CRISPR-Cas13a expression vector, pC003 (provided by Dr. F. Zhang), was amplified with pC003 PCR-r, "CCAGCTGTTAAACGAGCTTTAATGCGGTAGTTTATC", and pC003 PCR-f, "GAAGGGGACTAAAACGGAGACCGAGATTGGTCTCG", the regions of Cas13a, Cas1, and Cas2 were also amplified by LsCas13a clo-f, "TCGTTTAACAGCTGGGAAAATG", LsCas13a clo-r, "GTTTTAGTCCCCTTCGATATTGG" from the genomic DNA of Lepttrikia shahii (DSM 1975), two DNA fragments were bound by In-Fusion HD Cloning Kit (hereinafter, this is referred to as "pKLC5 vector"), the pKLC5 vector was cleaved with a BsaI restriction enzyme, and the crRNA sequence targeting bla_{IMP-1} was inserted. The target sequence is "ATGTTCATACTTCGTTTGAAGAAGTTAA" (complementary sequence) corresponding to SEQ ID NO: 17 in the sequence listing, and it is a region of 104 to 131 bases after the start of translation of bla_{IMP-1}. Two oligo DNAs ("tatccATGTTCATACTTCGTTTGAAGAAGTTAA", "aaacTTAACTTCTTCAAACGAAGTATGAACATg") were synthesized, annealed, and inserted into the BsaI site of pKLC5 ("pKLC5 BsaI IMP-1_104 vector", and hereinafter, referred to as "CRISPR-Cas13a expression vector").

### (The bactericidal effect of the CRISPR-Cas13a plasmid on bacteria having a specific gene)

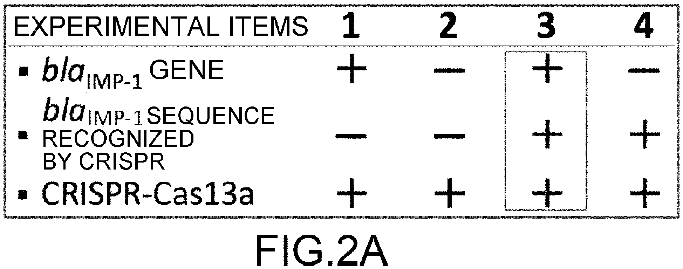

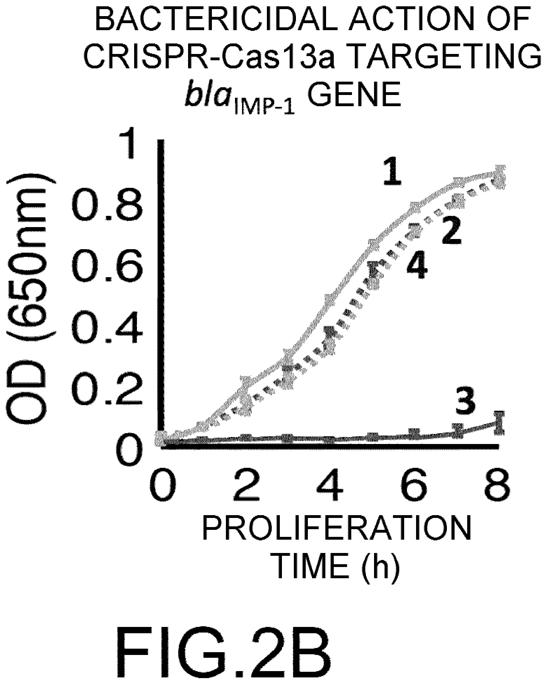

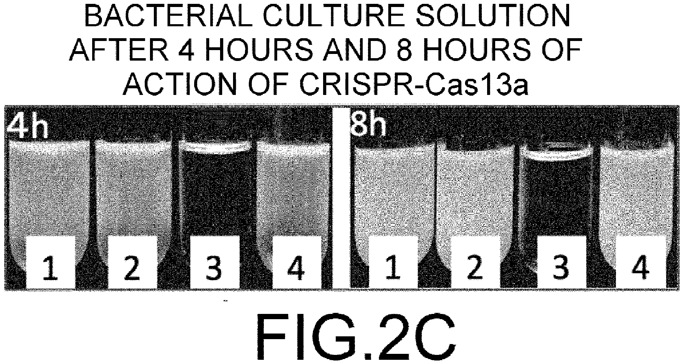

As refer to FIG. 2, the results were described by culturing the above-mentioned E. coli by transforming each plasmid of CRISPR-Cas13a of this example, transforming it, and culturing it. E. coli DH5-alpha strain was transformed with a tetracycline-induced bla_{IMP-1} expression vector (ampicillin resistance, ColE1 ori). This E. coli was further transformed with a CRISPR-Cas13a expression vector (chloramphenicol resistance, p15A ori) targeting the bla_{IMP-1} gene. The obtained E. coli was cultured in LB liquid medium (ampicillin, chloramphenicol) at 37 degree C for 12 hours, and then OD650 was adjusted to 0.02 in LB liquid medium (ampicillin, chloramphenicol). After shaking culture at 37 degree C for 1 hour, ice tetracycline was added to a final concentration of 100 ng / ml to induce the expression of the bla_{IMP-1} gene.

FIG. 2A shows the series of E. coli in which the experiment was conducted. Series 1, 2 and 4 show experimental examples of control, and series 3 shows experimental examples in which all are introduced. In the experimental item "bla_{IMP-1} sequence", "-" is E. coli into which only "pSP72 aTc-inducible vector" was introduced, and "+" was indicates E. coli into which the tetracycline-inducible bla_{Imp-1} expression vector was also introduced. The "bla_{IMP-1} sequence recognized by CRISPR" indicates that "-" is E. coli into which only pKLC5 has been introduced, and "+" is E. coli into which "CRISPR-Cas13a expression vector" has been introduced.

FIG. 2B is a graph showing the results of measuring OD650 over time. The horizontal axis represents the growth time (h) after the addition of ice tetracycline, and the vertical axis represents OD (650 nm).

FIG. 2C shows photographs of E. coli cultured in vitro after 4 hours (4h) and 8 hours (8h).

As shown in these results, CRISPR-Cas13a designed to recognize the bla_{IMP-1} gene as a target suppressed the growth of E. coli having the target specific gene bla_{IMP-1}. That is, it was confirmed that the designed CRISPR-Cas13a has a sequence-specific bactericidal effect on E. coli having the specific gene.

### [Bactericidal effect of antibacterial phage on bacteria having a specific gene]

Next, the designed CRISPR-Cas13a was mounted on a phage to verify the bactericidal effect on E. coli having a specific gene.

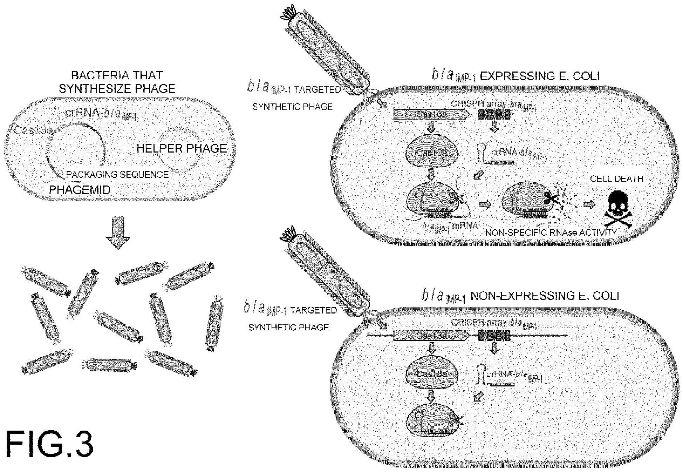

FIG. 3 shows a conceptual diagram of the synthesis of antibacterial phage, which is a synthetic phage carrying CRISPR-Cas13a targeting the bla_{Imp-1}, gene, and a conceptual diagram of the reaction when infected with the synthetic phage. Specifically, the phagemid and helper phage as described later are introduced into the phage-synthesizing bacterium and packaged, and thereby the antibacterial phage including CRISPR-Cas13a having a target sequence (hereinafter referred to as "bla_{IMP-1} targeted synthetic phage") is prepared. This bla_{IMP-1} targeted synthetic phage selectively kills E. coli expressing bla_{IMP-1}. On the other hand, the bla_{IMP-1} targeted synthetic phage does not kill E. coli that does not express bla_{IMP-1}.

### (Preparation of CRISPR-Cas13a expression vector targeting bla_{IMP-1})

The expression vector pKLC5 BsaI IMP-1_104 described above was improved as follows. Cas1 / 2 present on the vector was deleted because it does not participate in the bactericidal activity by Cas13a. This deletion is performed by performing PCR of the vector with Cas1Cas2 del SacI-f, "ATATGAGCTCATGGGAGAAAAAATTTCACAAAAC" and Cas1Cas2 del SacI-r, "ATATGAGCTCTCATTCTTATAACGTATCATTCG", after cleaving with SalI restriction enzyme, ligating with "Ligation high ver. 2" (manufactured by TOYOBO). Further, a work was carried out to confer f1 ori and kanamycin resistance to this vector. Specifically, as this vector was used as a template, PCR is performed with the primers, InF13 PCR SalI-f, "tcttcaccctgtcgatgggaaaatgtggaatttg", InF13 PCR SmaI-r, "caggatcttctgcccaattaggctctagttagcct", then, f1 ori and kanamycin resistance of pRC319 (obtained from Dr. Timothy Lu) were amplified with InF13 pRC319-f, "gggcagaagatcctgcagg", InF13 pRC319-r, "tcgacagggtgaagacgaaag", and two DNA fragments were bound by In-Fusion HD Cloning Kit (Hereinafter, this is referred to as "pKLC21 BsaI IMP-1_104 vector").

Further, as a control, a control vector obtained by similarly improving the expression vector pKLC5 was also prepared.

### (Preparation of M13 helper phage plasmid)

The helper phage M13KO7 (manufactured by NEB biolabs) was infected with E. coli NEB5-alpha F'l^{q} strain (manufactured by NEB biolabs), and the plasmid was recovered. The f1 origin sequence of M13KO7 was deleted to prevent self-packaging. By using the M13KO7 plasmid as a template, PCR was performed by using the primers of M13KO7 PCR In-Fusion-f, "cctattggttaaaaaatgagctg" and M13KO7 PCR In-Fusion-r, "actatggttgctttgacgag".

Further, p15A ori and chloramphenicol resistance gene of pBAD33 vector were amplified by pBAD33 PCR In-Fusion-f, "caaagcaaccatagtgtagcaccaggcgtttaagg" and pBAD33 PCR In-Fusion-r, "tttttaaccaataggcatcaccgatggggaagatc", and two amplified samples were combined with In-Fusion HD Cloning Kit (manufactured by TakaRa Bio) (hereinafter referred to as "pKLC25 vector").

### (Preparation of bla_{IMP-1} expressing E. coli (E. coli targeted by antibacterial phage))

To delete the extra sequence (f1 ori) of the pBAD33 vector, the pBAD33 vector was amplified with two primers, pBAD33 PCR XhoI-f, "tcaCTCGAGcgaatttgctttcgaatttc" and pBAD33 PCR XhoI-r, "tcaCTCGAGcaaaagagtttgtagaaacgc", after cleavage with the restriction enzyme XhoI, it was ligated by "Ligation high ver. 2" (hereinafter referred to as "pKLC23 vector"). The pKLC23 was amplified with two primers, pKLC23 PCR In-Fusion-f2, "GAATTCgatcctctagagtc" and pKLC23 PCR In-Fusion-r2, "tgcttcgtccatttgacag". Further, from the sequence of the native bla_{IMP-1} promoter sequence (artificial synthesis, manufactured by genewiz, GenBank ID: AB733642, 716bp to 1168bp), a part was amplified with Intl1 pro In-Fusion-f, "caaatggacgaagcaTGACGCACACCGTGGAAAC" and Intl1 pro In-Fusion-r, "tagaggatcGAATTCGAGAATGGATTTTGTGATGC". Then, two DNA fragments were bound by In-Fusion HD Cloning Kit (pKLC26 vector). The bla_{IMP-1} sequence is performed PCR amplification by using IMP-1 In-Fusion-f, "ACAAAATCCATTCTCATGAGCAAGTTATCTGTATTC" and IMP-1 In-Fusion-r, "tagaggatcGAATTCTTAGTTGCTTGGTTTTGATG", further, pKLC26 is used as a template, an amplified DNA fragment with primers of pKLC26 In-Fusion-f,"GAATTCgatcctctagagtc" and pKLC26 In-Fusion-r, "GAGAATGGATTTTGTGATGC" was prepared. The two DNA fragments were ligated by In-Fusion HD Cloning Kit (hereinafter referred to as "pKLC26 IMP-1 vector"). The E. coli NEB5-alpha F'l^{q} strain transformed with this pKLC26 IMP-1 vector (bla_{IMP-1} expressing E. coli) and E. coli NEB5-alpha F'l^{q} strain for control without transformation (bla_{IMP-1} non-expressing E. coli) were prepared.

### (Preparation of bla_{IMP-1} targeted synthetic phage)

Cas13a-loaded M13 phage (phagemid) targeting bla_{IMP-1} was generated. Specifically, the helper phage synthesis plasmid pKLC25 was transformed into E. coli MC1061 (phage synthesizing bacterium) and selected by chloramphenicol. This E. coli was transformed with a CRISPR-Cas13a expression vector (pKLC21 BsaI IMP-1_104 vector) targeting bla_{IMP-1} and was selected with chloramphenicol + kanamycin. The colonies were seismic cultured in LB liquid medium (chloramphenicol + kanamycin) at 37 degree C until E. coli were sufficiently increased. After centrifugation at 8,000 x g for 20 minutes, the supernatant was passed through a 0.22 micro-m filter. Add the same amount of PEG buffer (5mM Tris-HCl (pH 7.5), 10% PEG6000, 1M NaCl (58 g/L), 5 mM MgSO₄.7H₂O (1.23 g/L)), after mixing well, it was left at 4 degree C for 1 hour. Centrifuge at 4 degree C with 12,000 x g for 10 minutes to make pellets of antibacterial phage, eliminated the supernatant as much as possible, and then suspended in SM Buffer (50mM Tris-HCl pH7.5, 0.1M NaCl, 7mM MgSO₄.7H₂O, 0.01% gelatin). This was used as a solution of bla_{IMP-1} targeted synthetic phage.

Also, the control vector was also transformed in the same manner to prepare a solution of bla_{IMP-1} non-target synthetic phage.

### (Measurement of TFU (Transduced colonies forming unit))

These antibacterial phages (M13 phage carrying Cas13a) were diluted 1000-fold with SM Buffer. 1ml of E. coli NEB5-alpha F'l^{q} strain was previously liquid-cultured, and when the OD600 reached about 0.1, 10 micro-l of diluted antibacterial phage was added and the mixture was shaken at 37 degree C for 30 minutes. 100 micro-l of the bacterial solution was plated on an LB plate, an LB plate (containing chloramphenicol), and an LB plate (containing kanamycin). Then, they were incubated overnight at 37 degree C, after confirming that there was no E. coli on the LB plate (containing chloramphenicol), the number of colonies growing on the LB plate (containing kanamycin) was counted, and the TFU was calculated.

### (Phagemid induction assay)

The phagemid (M13 phage carrying Cas13a) on which TFU was measured was serially diluted 10-fold. When the pre-cultured E. coli NEB5-alpha F'l^{q} strain reaches about OD600 = 0.5, 100 micro-l of the bacterial solution and 3 mL of the LB solution (0.5% agarose) warmed to 50 degrees were mixed and poured into an LB plate. After the LB plate had hardened, 2 micro-l of each of the serially diluted antibacterial phage solution was added from above the plate and cultured at 37 degree C.

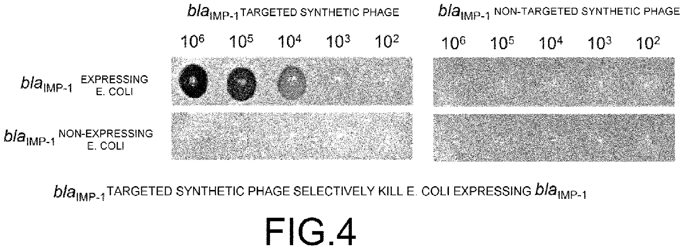

In FIG. 4, a solution of bla_{IMP-1} targeted synthetic phage or bla_{IMP-1} non-targeted synthetic phage was added to bla_{IMP-1} expressing E. coli or bla_{IMP-1} non-expressing E. coli at different concentrations. The result of whether or not it was lysed is shown. As a result, the bla_{IMP-1} targeted synthetic phage selectively killed E. coli expressing bla_{IMP-1}. That is, sequence-specific antibacterial activity was confirmed. In addition, the bla_{IMP-1} targeted synthetic phage showed a bactericidal effect even in a 1000-fold diluted solution.

### [Bacterial identification by using phage carrying CRISPR-Cas13a]

Next, we experimented with a bacteria identification method that can easily detect the bacteria where CRISPR-Cas13a is designed to target a specific gene to produce antibacterial phage loaded on M13 phage, and bacteria having the specific gene are selectively sterilized. The following is an example of the experiment. In this example, the resistance gene was targeted.

### (Inhibition of growth of specific gene-carrying E. coli by Cas13a)

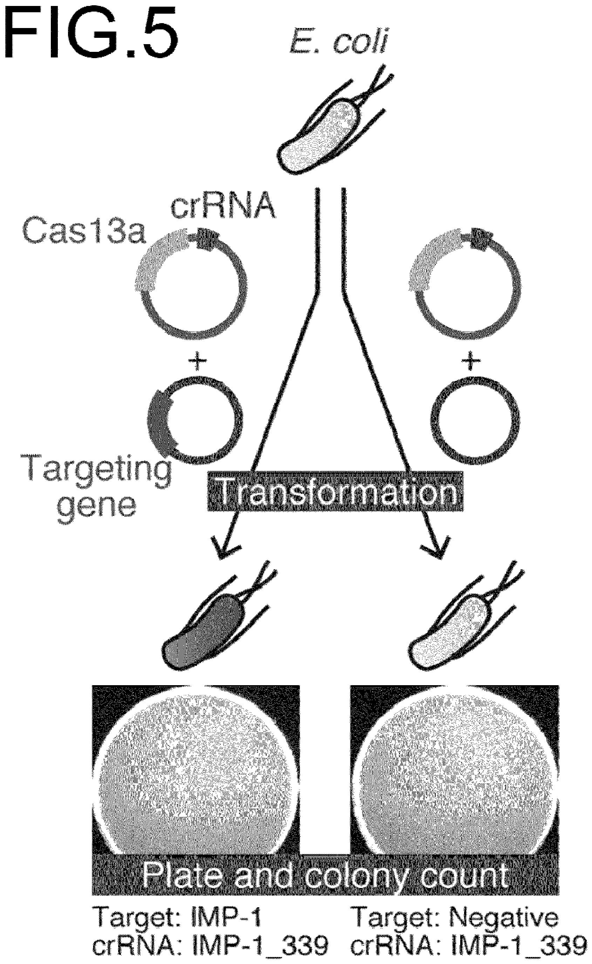

FIG. 5 shows an example of suppressing the growth of E. coli carrying a specific gene. E. coli strain MC1061 (manufactured by Lucigen, Inc.) was simultaneously transformed with i) a plasmid expressing Cas13a and crRNA and ii) a plasmid expressing a specific gene of crRNA (a plasmid expressing a targeting gene) . The plasmid of i) carries the kanamycin resistance gene, and the plasmid of ii) carries the chloramphenicol resistance gene. Transformed MC1061 was cultured on an LB plate (containing kanamycin and chloramphenicol), and a photograph of the plate was taken 12 hours later. When Cas13a and crRNA that recognizes the 339th base of IMP-1 was expressed in E. coli expressing IMP-1, the growth of E. coli was suppressed.

Preliminary experiments have shown that the bactericidal effect of CRISPR-Cas13a depends largely on the sequence structure of a particular gene. Therefore, the specificity (order) of the specific gene sequence of CRISPR-Cas13a was examined, and the optimum sequence was extracted. That is, in this example, the specific gene sequence of CRISPR-Cas13a was optimized.

In this example, with reference to the paper of CRISPR-Cas of Non-Patent Document 1, 3 to 8 base sequences for each specific gene were examined as the base sequence of the target site.

Specifically, target sequences of CRISPR-Cas13a were designed for 10 types of drug resistance genes. As these 10 kinds of genes, sequences of IMP-1 (GenBank ID:S71932), KPC-2 (AY034847), NDM-1 (FN396876), OXA-48 (AY236073), VIM-2 (AF191564), mcr-1 (KP347127), mcr-2 (LT598652), mcr-3 (KY924928), mcr-4 (MF543359), and mcr-5 (KY807921), are used. In addition, an RFP (Red Fluorescent Protein, GenBank ID: KJ021042) sequence was used as a control. Table 3 below shows the relationship between the SEQ ID NO: (No.) in the sequence listing, the name of the sequence mainly targeting the drug resistance gene (spacer name), and the selected target site base sequence (target sequence, spacer sequence).

**[Table 3]**

| No. | Spacer name | Spacer sequence |
|---|---|---|
| 1 | RFP | CGGTCTGGGTACCTTCGTACGGACGACCTTC |
| 2 | mcr-1_47 | AAAACGGCAACACTCGCCACAAGAACAA |
| 3 | mcr-1_1021 | CGGATTTATAATCGGCAAATTGCGCTTT |
| 4 | mcr-1_1495 | CCATTGGCGTGATGCCAGTTTGCTTATC |
| 5 | mcr-2_37 | CCACCAAACCCATCAGCACAAAAGGATT |
| 6 | mcr-2_1015 | CTGATTTATAATCAAAATACTGCGTGGC |
| 7 | mcr-2_1487 | GCAGTTGGCTTGAATGTCGTATTATTTG |
| 8 | mcr-3_34 | CAAAATACAGTGCCAAAAAGAACATAAG |
| 9 | mcr-3_997 | GGTGATCCTTTGGTTCGATTTCGATGTT |
| 10 | mcr-3_1436 | GTAAATCCAGGTGACATCCACACCTGCA |
| 11 | mcr-4_41 | GCAACATAAAACAACGCAGTGATGAAAG |
| 12 | mcr-4_1000 | GATCACTCTTCAAATCTATCGTGAGATT |
| 13 | mcr-4_1442 | CTAAAGTCATTAGATACCCAAGCCAGCA |
| 14 | mcr-5_50 | CTGATGAACAGAGTCAAAAATTCAGTGC |
| 15 | mcr-5_1024 | GATGGCCTGCCGAAGACAGGTTTTCAAA |
| 16 | mcr-5_1462 | CATAAACCTGACTCGACTGCCACCAGAT |
| 17 | IMP-1_104 | TTAACTTCTTCAAACGAAGTATGAACAT |
| 18 | IMP-1_291 | CCACTCTATTCCGCCCGTGCTGTCGCTA |
| 19 | IMP-1_298 | AATTAAGCCACTCTATTCCGCCCGTGCT |
| 20 | IMP-1_300 | AGAATTAAGCCACTCTATTCCGCCCGTG |
| 21 | IMP-1_331 | TTAATTCAGATGCATACGTGGGGATAGA |
| 22 | IMP-1_339 | TTCATTTGTTAATTCAGATGCATACGTG |
| 23 | IMP-1_436 | GGCCTGGATAAAAAACTTCAATTTTATT |
| 24 | IMP-1_615 | ACTTGGAACAACCAGTTTTGCCTTACCA |
| 25 | NDM-1_117 | ATCGCCAAACCGTTGGTCGCCAGTTTCC |
| 26 | NDM-1_406 | ACAACGCATTGGCATAAGTCGCAATCCC |
| 27 | NDM-1_724 | AATGGCTCATCACGATCATGCTGGCCTT |
| 28 | OXA-48_201 | ATTGGGAATTTTAAAGGTAGATGCGGGT |
| 29 | OXA-48_364 | CAATTTGGCGGGCAAATTCTTGATAAAC |
| 30 | OXA-48_470 | GCCGAAATTCGAATACCACCGTCGAGCC |
| 31 | KPC-2_50 | GTCAGCGCGGTGGCAGAAAAGCCAGCCA |
| 32 | KPC-2_232 | CCTGCTGCTGGCTGCGAGCCAGCACAGC |
| 33 | KPC-2_732 | CCCAGTGGGCCAGACGACGGCATAGTCA |
| 34 | VIM-2_229 | CTGTATCAATCAAAAGCAACTCATCACC |
| 35 | VIM-2_335 | CCGACGCGGTCGTCATGAAAGTGCGTGG |
| 36 | VIM-2_518 | TCGGTCGAATGCGCAGCACCAGGATAGA |

The bactericidal effect of CRISPR-Cas13a was evaluated by designing crRNA against these target site base sequences.

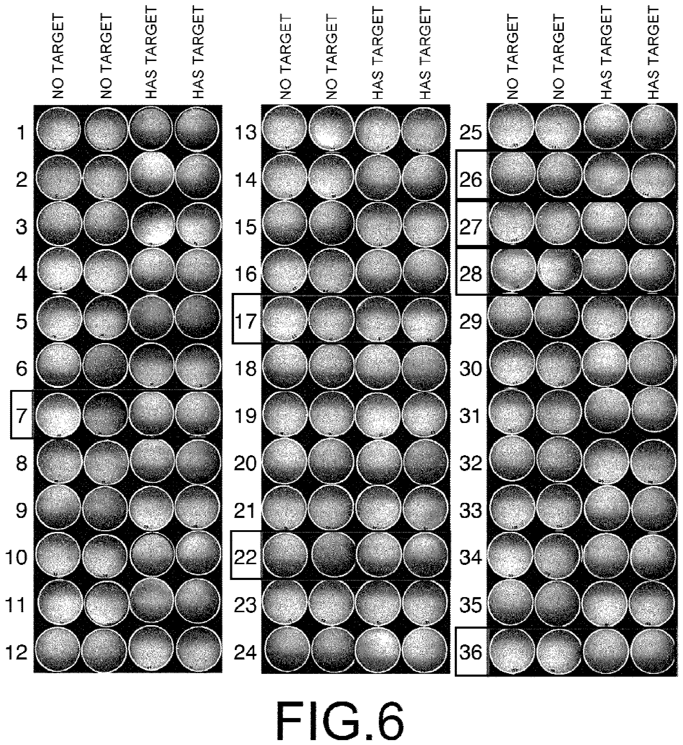

The photograph of FIG. 6 shows the results of designing crRNA for each target sequence in Table 3 and evaluating the bactericidal effect of CRISPR-Cas13a by using it. Specifically, E. coli MC1061 was simultaneously transformed with i) Cas13a, a plasmid expressing crRNA corresponding to each target site base sequence, and ii) a plasmid expressing a specific gene of crRNA (with target). The plasmid of i) carries the kanamycin resistance gene, and the plasmid of ii) carries the chloramphenicol resistance gene. FIG. 6 shows a photograph of plates taken of the transformed MC1061 after culturing on LB plates (containing kanamycin and chloramphenicol) cultured at 37 degree C for 12 hours. About each sequence number, for no crDNA (no target) for the target sequence, from the left, a plate of untransformed (without drug resistance gene) and transformed (with drug resistance gene) are shown. Also, regarding crDNA (with target), from the left, the plates for bacteria without drug resistance genes and bacteria with drug resistance genes are shown. In the plate with the sequence number surrounded by a black frame, in E. coli plates with targets and drug resistance genes, almost no colonies appeared, and strong bactericidal activity (growth inhibitory activity) by Cas13a was confirmed. As described above, the bactericidal effect was different depending on the target sequence. Further, strong bactericidal activity (proliferation inhibitory activity) was confirmed when the crDNA having each of SEQ ID NO: 7, SEQ ID NO: 17, SEQ ID NO: 22, SEQ ID NO: 26, SEQ ID NO: 27, SEQ ID NO: 28, and SEQ ID NO: 36 as the target sequence was introduced.

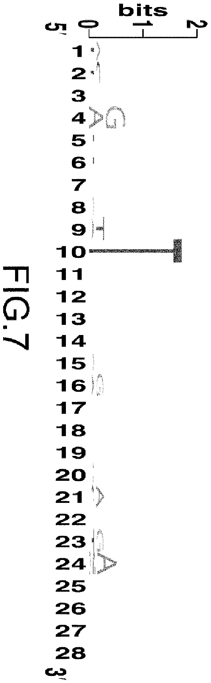

FIG. 7 shows that the target sequences of the sequence numbers for the plates shown in the black frame in FIG. 6 are aligned by Weblogo (URL <https: //weblogo.berkeley.edu/>). A bias is seen, as represented by the 10th T. That is, as a result of the examination, in the target sequence designed as a spacer sequence of crRNA of 14 to 28 bases, when the target at the 10th base was T, the bactericidal activity of CRISPR-Cas13a was optimal.

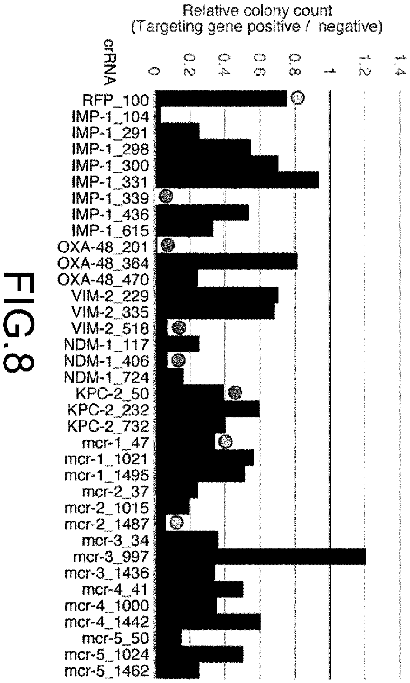

FIG. 8 is a graph showing the visually counted number of colonies on the plate of the experiment performed in FIG. 6. However, small colonies are excluded from the count. Here, for each drug resistance gene plate, a relative colony count that the number of colonies on the E. coli plate with the drug resistance gene (with the resistance gene) was divided by the number of colonies on the E. coli plate without the drug resistance gene (without the resistance gene) was shown. All crRNAs other than mcr-3_997 inhibited the growth of E. coli carrying the target drug resistance gene. On this, the part indicated by the circle showed recognizing the above-mentioned strong bactericidal activity and the control RFP (RFP_100). In this way, it is shown that the resistant gene-carrying bacteria can be sterilized efficiently.

### (Identification of E. coli carbapenem resistance gene by antibacterial phage)

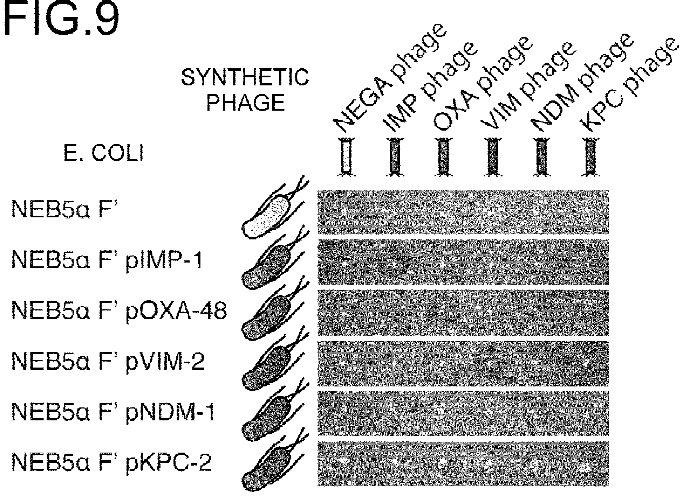

FIG. 9 shows the results of identifying the carbapenem resistance gene of E. coli with the Cas13a-loaded antibacterial phage. In this experiment, M13 phage was loaded with crRNA and Cas13a for the target sequence in which the above-mentioned strong bactericidal activity was confirmed. E. coli, which is the E. coli NEB5-alpha F'l^{q} strain, was transformed by using each of plasmids of IMP-1, OXA-48, VIM-2, NDM-1, and KPC-2 for carbapenem resistance genes. The prepared E. coli was infected with an antibacterial phage prepared by loading crRNA and Cas13a of each target sequence in the same manner as the above-mentioned bla_{IMP-1} targeted synthetic phage, incubated on LB soft agar medium without antibiotics, and confirmed that the growth of E. coli was inhibited. That is, when E. coli having each resistance gene was infected with each antibacterial phage, its growth was suppressed. In this way, it could be used to identify (discriminate) whether E. coli had the carbapenem resistance gene.

### (Identification of E. coli colistin resistance gene by antibacterial phage)

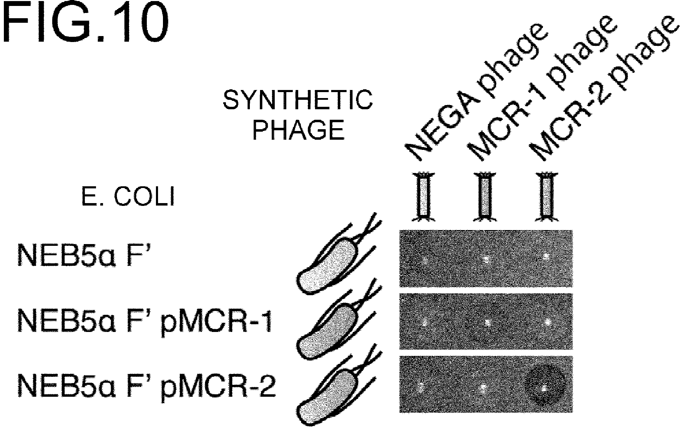

FIG. 10 shows the results of identifying the colistin resistance gene of E. coli with the Cas13a-loaded antibacterial phage. In this experiment, M13 phage was loaded with crRNA and Cas13a for the target sequence in which the above-mentioned strong bactericidal activity was confirmed. E. coli of NEB 5-alpha F'll^{q} strain was transformed with MCR-1 and MCR-2 plasmids as colistin resistance genes. The prepared E. coli was infected with the antibacterial phage prepared in the same manner as described above, incubated on LB soft agar medium without antibiotics, and inhibition of E. coli growth was confirmed. Again, it was confirmed that growth was suppressed when each antibacterial phage was infected with E. coli having each resistance gene. That is, it was possible to use it for identifying (discriminating) whether E. coli carries the colistin resistance gene.

### [Example 2]

### (Modification of E. coli flora by antibacterial phage)

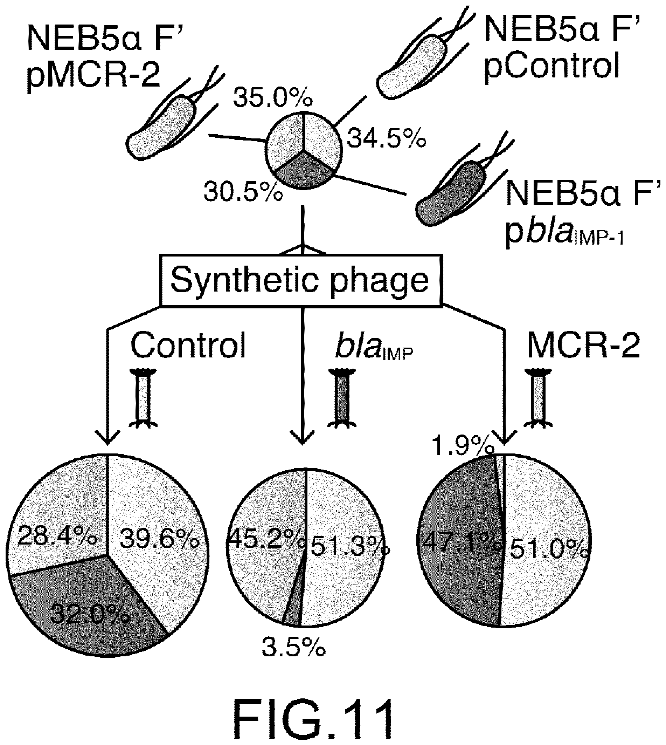

FIG. 11 shows the results of selectively eliminating bacteria carrying the carbapenem resistance gene bla_{IMP-1} and colistin resistance gene MCR-2 of E. coli with the antibacterial phage loaded Cas13a.

In this example, M13 phage was loaded with crRNA and Cas13a for the target sequence in which strong bactericidal activity was confirmed in the above Example 1. E. coli of the NEB 5-alpha F'l^{q} strain was transformed with an expression plasmid of the carbapenem resistance gene bla_{IMP-1} and the colistin resistance gene MCR-2. The prepared E. coli was infected with the antibacterial phage prepared in the same manner as in Example 1 and cultured for 6 hours. They were cultured on LB soft agar medium without antibiotics to create a dilution series of liquids. Each sample was plated on an LB plate, an ampicillin-containing LB plate, and a colistin-containing LB plate. After culturing all the plates at 37 degree C for 12 hours, the number of colonies growing on the plates was counted. Bacteria grown on the ampicillin-containing LB plate were used as the bla_{IMP-1} expressing strain, bacteria growing on the colistin-containing LB plate were used as the MCR-2 expressing strain, and other bacteria were used as the control strains, and the ratio of each strain was calculated.

The antibacterial phage targeting bla_{IMP-1} reduced the number of bla_{IMP-1} expression strains, and the antibacterial phage targeting MCR-2 reduced the number of MCR-2 expression strains, respectively. That is, the Cas13a-loaded antibacterial phage could be used to reduce E. coli carrying a specific gene.

### (Optimization of CRISPR-Cas13a expression vector targeting the bla_{IMP-1} gene)

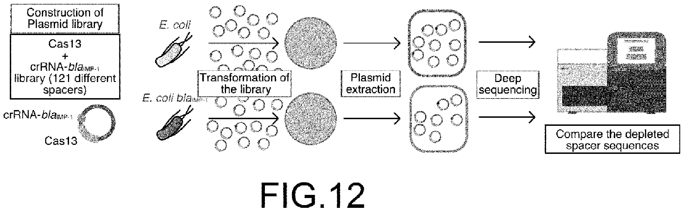

FIG. 12 is a conceptual diagram of a method for investigating the optimum CRISPR sequence for the bactericidal effect of a bacterium having the bla_{IMP-1} gene by CRISPR-Cas13a.

We have prepared 121 kinds of different spacers targeting bla_{IMP-1} were inserted into pKLC21.

Table 4 below shows the relationship between the SEQ ID NO: (No.) of the sequence listing, the name of the sequence for the bla_{IMP-1} gene (Spacer name), and the selected target site base sequence (Target Sequence, Spacer Sequence).

**[Table 4]**

| No. | Spacer name | Spacer sequence | No. | Spacer name | Spacer sequence |
|---|---|---|---|---|---|
| 1 | IMP-1_11 | CAAAACAAAAATATAAAGAATACAGATA | 62 | IMP-1_363 | AACCTTACCGTCTTTTTTAAGCAGTTCA |
| 2 | IMP-1_14 | CTGCAAAACAAAAATATAAAGAATACAG | 63 | IMP-1_367 | CTTGAACCTTACCGTCTTTTTTAAGCAG |
| 3 | IMP-1_19 | CAATGCTGCAAAACAAAAATATAAAGAA | 64 | IMP-1_368 | GCTTGAACCTTACCGTCTTTTTTAAGCA |
| 4 | IMP-1_22 | TAGCAATGCTGCAAAACAAAAATATAAA | 65 | IMP-1_370 | TGGCTTGAACCTTACCGTCTTTTTTAAG |
| 5 | IMP-1_23 | GTAGCAATGCTGCAAAACAAAAATATAA | 66 | IMP-1_376 | AATTTGTGGCTTGAACCTTACCGTCTTT |
| 6 | IMP-1_25 | CGGTAGCAATGCTGCAAAACAAAAATAT | 67 | IMP-1_383 | CTAAATGAATTTGTGGCTTGAACCTTAC |
| 7 | IMP-1_28 | CTGCGGTAGCAATGCTGCAAAACAAAAA | 68 | IMP-1_392 | TTAACTCCGCTAAATGAATTTGTGGCTT |
| 8 | IMP-1_34 | ACTCTGCTGCGGTAGCAATGCTGCAAAA | 69 | IMP-1_399 | CCAATAGTTAACTCCGCTAAATGAATTT |
| 9 | IMP-1_39 | CAAAGACTCTGCTGCGGTAGCAATGCTG | 70 | IMP-1_429 | ATAAAAAACTTCAATTTTATTTTTAACT |
| 10 | IMP-1_40 | GCAAAGACTCTGCTGCGGTAGCAATGCT | 71 | IMP-1_430 | GATAAAAAACTTCAATTTTATTTTTAAC |
| 11 | IMP-1_60 | CTTTTCAATTTTTAAATCTGGCAAAGAC | 72 | IMP-1_434 | CCTGGATAAAAAACTTCAATTTTATTTT |
| 12 | IMP-1_71 | CCTTCATCAAGCTTTTCAATTTTTAAAT | 73 | IMP-1_435 | GCCTGGATAAAAAACTTCAATTTTATTT |
| 13 | IMP-1_81 | AACATAAACGCCTTCATCAAGCTTTTCA | 74 | IMP-1_436 | GGCCTGGATAAAAAACTTCAATTTTATT |
| 14 | IMP-1_85 | TATGAACATAAACGCCTTCATCAAGCTT | 75 | IMP-1_441 | TCCCGGGCCTGGATAAAAAACTTCAATT |
| 15 | IMP-1_88 | AAGTATGAACATAAACGCCTTCATCAAG | 76 | IMP-1_443 | TGTCCCGGGCCTGGATAAAAAACTTCAA |
| 16 | IMP-1_104 | TTAACTTCTTCAAACGAAGTATGAACAT | 77 | IMP-1_445 | TGTGTCCCGGGCCTGGATAAAAAACTTC |
| 17 | IMP-1_113 | CCCCACCCGTTAACTTCTTCAAACGAAG | 78 | IMP-1_447 | AGTGTGTCCCGGGCCTGGATAAAAAACT |
| 18 | IMP-1_120 | AACAACGCCCCACCCGTTAACTTCTTCA | 79 | IMP-1_448 | GAGTGTGTCCCGGGCCTGGATAAAAAAC |
| 19 | IMP-1_121 | GAACAACGCCCCACCCGTTAACTTCTTC | 80 | IMP-1_455 | TTATCTGGAGTGTGTCCCGGGCCTGGAT |
| 20 | IMP-1_126 | TTTAGGAACAACGCCCCACCCGTTAACT | 81 | IMP-1_471 | CAACCAAACCACTACGTTATCTGGAGTG |
| 21 | IMP-1_141 | AACCACCAAACCATGTTTAGGAACAACG | 82 | IMP-1_472 | GCAACCAAACCACTACGTTATCTGGAGT |
| 22 | IMP-1_151 | CATTTACAAGAACCACCAAACCATGTTT | 83 | IMP-1_491 | AATAATATTTTCCTTTCAGGCAACCAAA |
| 23 | IMP-1_157 | CCTCAGCATTTACAAGAACCACCAAACC | 84 | IMP-1_510 | TTTAATAAAACAACCACCGAATAATATT |
| 24 | IMP-1_161 | TAAGCCTCAGCATTTACAAGAACCACCA | 85 | IMP-1_511 | GTTTAATAAAACAACCACCGAATAATAT |
| 25 | IMP-1_162 | GTAAGCCTCAGCATTTACAAGAACCACC | 86 | IMP-1_515 | TACGGTTTAATAAAACAACCACCGAATA |
| 26 | IMP-1_170 | TCAATTAGGTAAGCCTCAGCATTTACAA | 87 | IMP-1_524 | CCTAAACCGTACGGTTTAATAAAACAAC |
| 27 | IMP-1_172 | TGTCAATTAGGTAAGCCTCAGCATTTAC | 88 | IMP-1_536 | TCACCCAAATTGCCTAAACCGTACGGTT |
| 28 | IMP-1_174 | AGTGTCAATTAGGTAAGCCTCAGCATTT | 89 | IMP-1_538 | CGTCACCCAAATTGCCTAAACCGTACGG |
| 29 | IMP-1_175 | GAGTGTCAATTAGGTAAGCCTCAGCATT | 90 | IMP-1_550 | CTTCTATATTTGCGTCACCCAAATTGCC |
| 30 | IMP-1_181 | TAAATGGAGTGTCAATTAGGTAAGCCTC | 91 | IMP-1_555 | CCAAGCTTCTATATTTGCGTCACCCAAA |
| 31 | IMP-1_184 | CCGTAAATGGAGTGTCAATTAGGTAAGC | 92 | IMP-1_556 | GCCAAGCTTCTATATTTGCGTCACCCAA |
| 32 | IMP-1_193 | TATCTTTAGCCGTAAATGGAGTGTCAAT | 93 | IMP-1_562 | ACTTTGGCCAAGCTTCTATATTTGCGTC |
| 33 | IMP-1_206 | ACTAACTTTTCAGTATCTTTAGCCGTAA | 94 | IMP-1_563 | GACTTTGGCCAAGCTTCTATATTTGCGT |
| 34 | IMP-1_208 | TGACTAACTTTTCAGTATCTTTAGCCGT | 95 | IMP-1_565 | CGGACTTTGGCCAAGCTTCTATATTTGC |
| 35 | IMP-1_222 | CTCCACAAACCAAGTGACTAACTTTTCA | 96 | IMP-1_566 | GCGGACTTTGGCCAAGCTTCTATATTTG |
| 36 | IMP-1_227 | CCACGCTCCACAAACCAAGTGACTAACT | 97 | IMP-1_580 | ACTTTAATAATTTGGCGGACTTTGGCCA |
| 37 | IMP-1_242 | CCTTTTATTTTATAGCCACGCTCCACAA | 98 | IMP-1_581 | GACTTTAATAATTTGGCGGACTTTGGCC |
| 38 | IMP-1_245 | CTGCCTTTTATTTTATAGCCACGCTCCA | 99 | IMP-1_595 | CCTTACCATATTTGGACTTTAATAATTT |
| 39 | IMP-1_250 | AAATGCTGCCTTTTATTTTATAGCCACG | 100 | IMP-1_600 | TTTTGCCTTACCATATTTGGACTTTAAT |
| 40 | IMP-1_251 | GAAATGCTGCCTTTTATTTTATAGCCAC | 101 | IMP-1_609 | AACAACCAGTTTTGCCTTACCATATTTG |
| 41 | IMP-1_253 | AGGAAATGCTGCCTTTTATTTTATAGCC | 102 | IMP-1_610 | GAACAACCAGTTTTGCCTTACCATATTT |
| 42 | IMP-1_255 | AGAGGAAATGCTGCCTTTTATTTTATAG | 103 | IMP-1_615 | ACTTGGAACAACCAGTTTTGCCTTACCA |
| 43 | IMP-1_261 | AAAATGAGAGGAAATGCTGCCTTTTATT | 104 | IMP-1_617 | TGACTTGGAACAACCAGTTTTGCCTTAC |
| 44 | IMP-1_266 | CTATGAAAATGAGAGGAAATGCTGCCTT | 105 | IMP-1_632 | TCTCCAACTTCACTGTGACTTGGAACAA |
| 45 | IMP-1_269 | TCGCTATGAAAATGAGAGGAAATGCTGC | 106 | IMP-1_634 | CGTCTCCAACTTCACTGTGACTTGGAAC |
| 46 | IMP-1_272 | CTGTCGCTATGAAAATGAGAGGAAATGC | 107 | IMP-1_637 | ATGCGTCTCCAACTTCACTGTGACTTGG |
| 47 | IMP-1_274 | TGCTGTCGCTATGAAAATGAGAGGAAAT | 108 | IMP-1_639 | TGATGCGTCTCCAACTTCACTGTGACTT |
| 48 | IMP-1_278 | CCCGTGCTGTCGCTATGAAAATGAGAGG | 109 | IMP-1_641 | AGTGATGCGTCTCCAACTTCACTGTGAC |
| 49 | IMP-1_291 | CCACTCTATTCCGCCCGTGCTGTCGCTA | 110 | IMP-1_648 | TTTCAAGAGTGATGCGTCTCCAACTTCA |
| 50 | IMP-1_298 | AATTAAGCCACTCTATTCCGCCCGTGCT | 111 | IMP-1_652 | TAAGTTTCAAGAGTGATGCGTCTCCAAC |
| 51 | IMP-1_300 | AGAATTAAGCCACTCTATTCCGCCCGTG | 112 | IMP-1_660 | CTCTAATGTAAGTTTCAAGAGTGATGCG |
| 52 | IMP-1_304 | ATCGAGAATTAAGCCACTCTATTCCGCC | 113 | IMP-1_664 | CCTGCTCTAATGTAAGTTTCAAGAGTGA |
| 53 | IMP-1_308 | ATAGATCGAGAATTAAGCCACTCTATTC | 114 | IMP-1_680 | TTTAACCCTTTAACCGCCTGCTCTAATG |
| 54 | IMP-1_309 | GATAGATCGAGAATTAAGCCACTCTATT | 115 | IMP-1_693 | TTTTTTACTTTCGTTTAACCCTTTAACC |
| 55 | IMP-1_310 | GGATAGATCGAGAATTAAGCCACTCTAT | 116 | IMP-1_694 | GTTTTTTACTTTCGTTTAACCCTTTAAC |
| 56 | IMP-1_311 | GGGATAGATCGAGAATTAAGCCACTCTA | 117 | IMP-1_697 | ATGGTTTTTTACTTTCGTTTAACCCTTT |
| 57 | IMP-1_322 | ATGCATACGTGGGGATAGATCGAGAATT | 118 | IMP-1_702 | TTTTGATGGTTTTTTACTTTCGTTTAAC |
| 58 | IMP-1_331 | TTAATTCAGATGCATACGTGGGGATAGA | 119 | IMP-1_703 | GTTTTGATGGTTTTTTACTTTCGTTTAA |
| 59 | IMP-1_339 | TTCATTTGTTAATTCAGATGCATACGTG | 120 | IMP-1_707 | CTTGGTTTTGATGGTTTTTTACTTTCGT |
| 60 | IMP-1_342 | CAGTTCATTTGTTAATTCAGATGCATAC | 121 | IMP-1_710 | TTGCTTGGTTTTGATGGTTTTTTACTTT |
| 61 | IMP-1_353 | TCTTTTTTAAGCAGTTCATTTGTTAATT | | | |

The above-mentioned 121 kinds of plasmid vectors were mixed in equal amounts to prepare a plasmid library. This plasmid library used to transform E. coli MC1061 pKLC26 or bMC1061 pKLC26 bla_{IMP-1}, and each of them was plated on twenty LB plates (containing chloramphenicol and kanamycin). After culturing at 37 degree C for 16 hours, it was confirmed that the number of colonies was 10,000 or more in each plate group, 5 ml of medium was added to the plates, and all colonies were collected with a scraper. After centrifuging the bacterium, the plasmid was extracted by using QIAGEN Plasmid Midi Kit (manufactured by QIAGEN), and the nucleotide sequence was sequenced by Illumina MiSeq. A plasmid library of mate-pair was prepared by using the Nextera mate-pair sample preparation kit (manufactured by Illumina, Inc., San Diego, CA, USA), and the plasmid library was sequenced by MiSeq reagent kit version 3 (Illumina, Inc.). After that, the number of each spacer sequence was counted.

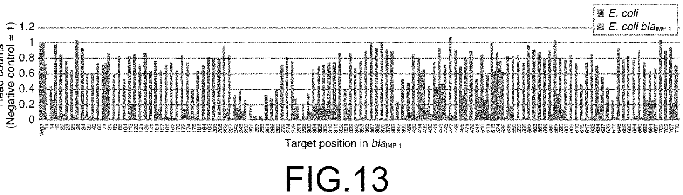

FIG. 13 is a diagram showing the bactericidal effect of CRISPR-Cas13a on which 121 types of bla_{IMP-1} spacer sequences are separately loaded on E. coli MC1061 having the bla_{IMP-1} gene. When the spacer sequences are No. 563 (GACTTTGGCCAAGCTTCTATATTTGCGT), No. 566 (GCGGACTTTGGCCAAGCTTCTATATTTG), No. 702 (TTTTGATGGTTTTTTACTTTCGTTTAAC), No. 562 (ACTTTGGCCAAGCTTCTATATTTGCGTC), No. 370 (TGGCTTGAACCTTACCGTCTTTTTTAAG), No. 565 (CGGACTTTGGCCAAGCTTCTATATTTGC), No. 193 (TATCTTTAGCCGTAAATGGAGTGTCAAT), No. 274 (TGCTGTCGCTATGAAAATGAGAGGAAAT), No. 648 (TTTCAAGAGTGATGCGTCTCCAACTTCA), No. 11 (CAAAACAAAAATATAAAGAATACAGATA), No. 609 (AACAACCAGTTTTGCCTTACCATATTTG), No. 703 (GTTTTGATGGTTTTTTACTTTCGTTTAA), and No. 208 (TGACTAACTTTTCAGTATCTTTAGCCGT), the sequence count of the bla_{IMP-1} having strain was 100 times or more lower than that of the bla_{IMP-1} non-having strain. In the following experiment, the spacer sequence targeting bla_{IMP-1} is used No. 563, which has the highest inhibitory effect.

### (Method of increasing gene detection sensitivity of antibacterial phage)

Next, in order to increase the gene detection sensitivity of the antibacterial phage, the present inventors conducted an experiment in which the antibacterial phage included an antibiotic resistance gene to detect the gene.

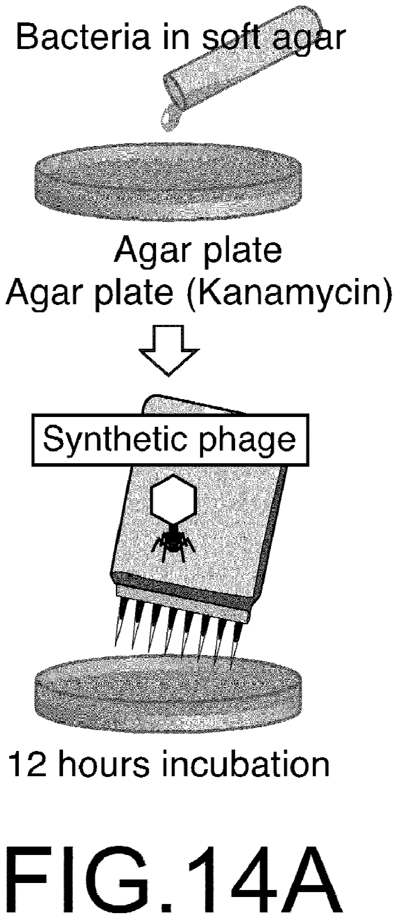

FIG. 14A shows a conceptual diagram how to increase the sensitivity of gene detection of antibacterial phage. M13-based antibacterial phage carrying the CRISPR-Cas13a sequence and the kanamycin resistance gene sequence was synthesized. A detection experiment was carried out by using an agar plate containing no kanamycin (Agar plate) and an agar plate containing kanamycin (Agar plate (Kanamycin)). The product of a mixture of the target bacterium (NEB5-alpha F'l^{q} pKLC26 bla_{IMP-1}) and soft agar medium was poured and left to be hardened. Immediately after the plate had hardened, antibacterial phage was added from above, and the bacteria were cultured at 37 degree C for 12 hours.

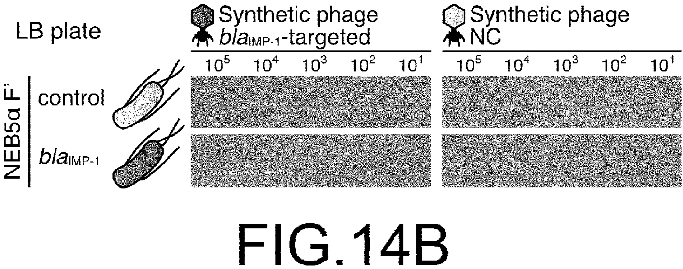

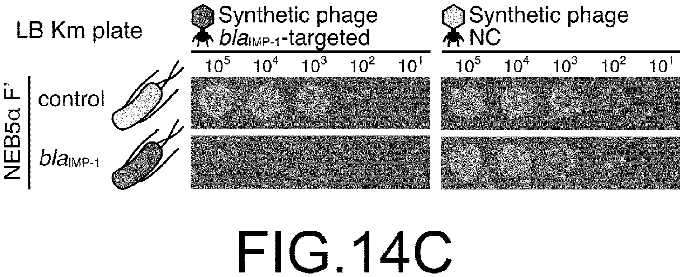

FIG. 14B shows the experimental results on the kanamycin-free agar plate, and FIG. 14C shows the experimental results on the kanamycin-containing agar plate. Regarding the control (strain not carrying bla_{IMP-1}) and the strain carrying bla_{IMP-1}, respectively, photographs of the results for each TFU are shown for those with the target gene introduced (bla_{IMP-1}-targeted) and those without the target gene (NC).

In this experiment, when cultured in an agar plate containing no antibiotic (agar plate containing no antibiotic) for the resistance gene possessed by the phage as in Example 1, for detection, it was necessary to use antibacterial phage in the range of 10⁴ TFU to 10⁵ TFU.

On the other hand, it was confirmed that when the antibacterial phage had an antibiotic resistance gene, it can be sufficiently detected even at about 10³ TFU. That is, the detection sensitivity of the gene could be increased about 100 times. Since this method only causes the antibacterial phage to carry the resistance gene, it can be applied not only to M13 phage but also to all other antibacterial phage.

### (Identification of carbapenem resistance gene by antibacterial phage)

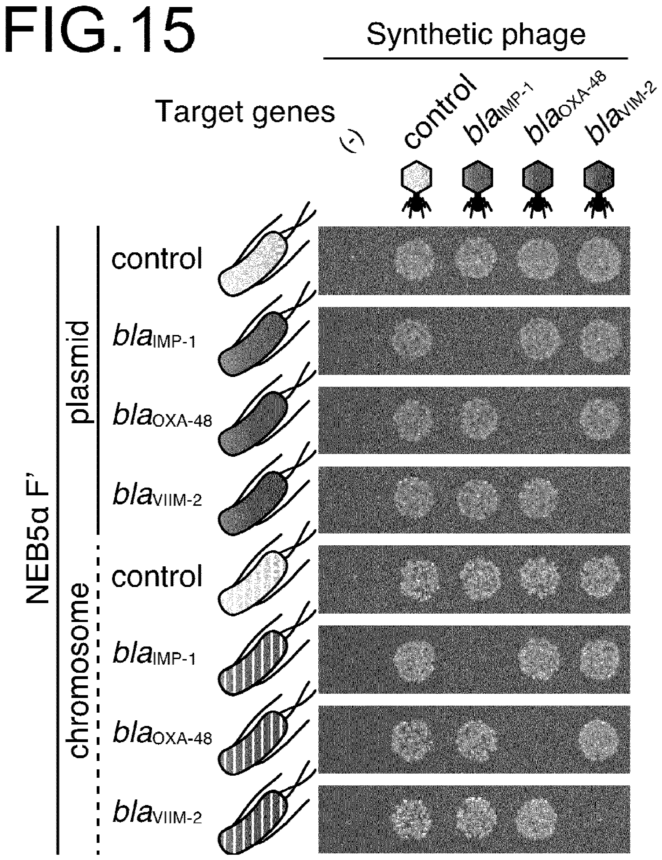

FIG. 15 is a photograph in which the carbapenem resistance genes bla_{IMP-1}, bla_{OXA-48}, and bla_{VIM-2} were identified by using the method of FIG. 14. The E. coli NEB5-alpha F'l^{q} expressing a resistance gene from the pKLC26 plasmid (plasmid) or NEB5-alpha F'l^{q} expressing the resistance gene from the genome (chromosome) were prepared, 10⁴ TFU antibacterial phage were added in the same manner as in FIG. 14, and the result of culturing at 37 degree C for 12 hours were shown.

For each, the results for the control having no carbapenem resistance gene, for bla_{IMP-1}, bla_{OXA-48}, and bla_{VIM-2}, for which the target gene was added, and for the negative control (-) of adding only water were shown. As a result, the bacteria having each gene are selectively lysed can be seen.

The method for expressing the resistance gene on the chromosome of the E. coli NEB5-alpha F'l^{q} strain was as follows. NEB5-alpha F'l^{q} was transformed by PKD46 into and selectively cultured on an ampicillin-containing LB plate. Also, the drug resistance gene encoded by pKLC26 vector was amplified by the primers, K12 genome-in pKLC26-f, "CCCTTCAACCTTAGCAGTAGCGTGGGATGATTTCACAATTAGAAAGACCTTGACGCACACCG TGGAAAC" and K12 genome-in pKLC26Cm-r, "TGTCCTGCACGACGCCTTGCGTCACTAGCCTCTTCTCTAGCTCATCATGCTGAGACGTTGAT CGGCACG". The amplified DNA was designed to include the target drug resistance gene and the chloramphenicol resistance gene, and the insertion site was designed in a non-coding region that is not conserved between species. The NEB5-alpha F'l^{q} pKD46 strain or MC1061 pKD46 strain was cultured in an ampicillin-containing LB medium, and when the OD600 reached 0.2, L-arabinose was added to a final concentration of 0.3%, and the bacteria were vigorously shake-cultured for 15 minutes at 42 degree C. After washing 3 times with ice-cold 10% glycerol, 100 ng of amplified DNA fragments (having homologous sequences to E. coli at the 5'- and 3'- ends) are transferred to an ice-cooled 1 mm cuvette and electroporated with ELEP021 (Poring Pulse (Voltage: 2,000 V, Pulse width: 2.5 msec, Pulse interval: 50 ms, Number of times: 1, Polarity: +), Transfer Pulse (Voltage: 150 V, Pulse width: 50 msec, Pulse interval: 50 ms, Number of times: 5, Polarity: +/-)) was performed. Immediately, 1 mL SOC was added, and the mixture was shake-cultured at 37 degree C, and then, it was selectively cultured on an LB plate containing chloramphenicol. The sequence around the modification of the genetically modified E. coli, which was produced here, was confirmed by sequencing of the Sanger method.

### (Identification of toxin genes by antibacterial phage)

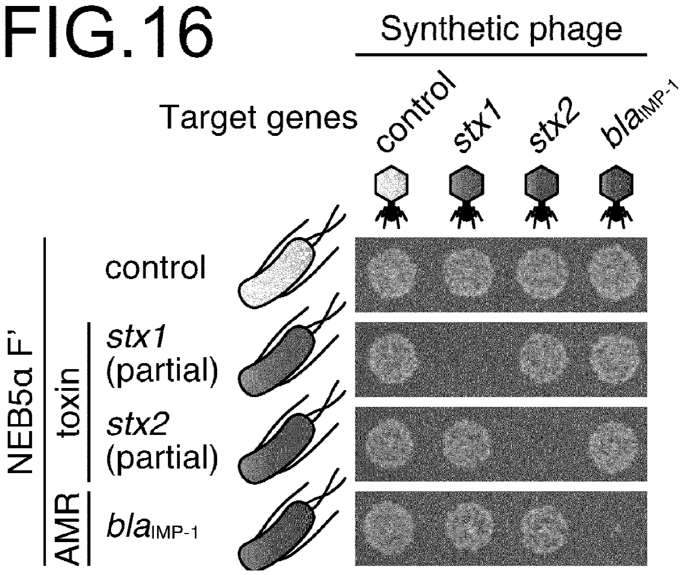

FIG. 16 is a photograph in which the toxin genes stx1 and stx2 were identified by using antibacterial phage. Specifically, the control and each toxin gene (toxin) stx1 and stx2, and resistance gene (AMR) could be detected by antibacterial phage.

Note that stx1 and stx2 use a part of the arrangement, not the total length. It is amplified from the pKLC26 plasmid with primers, pKLC26 stx1 partial PCR SacI-r, "GTGGAGCTCGGTCATGGCATTTCCACTAAACTCCATTAAGAGAATGGATTTTGTGATGC", pKLC26 stx1 partial PCR SacI -f, "ACCGAGCTCCACCGGAAGAAGTGGAACTCACACTGAACGAATTCGATCCTCTAGAGTC", pKLC26 stx2 partial PCR SacI -r, "GCGGAGCTCGGTCATTGTATTACCACTGAACTCCATTAAGAGAATGGATTTTGTGATGC", and pKLC26 stx2 partial PCR SacI -f, "ACCGAGCTCCGCCGGGAGACGTGGACCTCACTCTGAACGAATTCGATCCTCTAGAGTC", and after processing with SacI (manufactured by TakaRa Bio), it is ligated by using "Ligation High Ver. 2" (manufactured by TOYOBO). (Hereinafter, each is referred to as "pKLC26 stx1 vector" and "pKLC26 stx2 vector").

In addition, a double-stranded DNA annealed with stx1 488-as, "TATCCTAATGGAGTTTAGTGGAAATGCCATGAC" and stx1 488-s, "AAACGTCATGGCATTTCCACTAAACTCCATTAG" or a double-stranded DNA annealed with stx2 488-as, "TATCCTAATGGAGTTCAGTGGTAATACAATGAC" and stx2 488-s, "AAACGTCATTGTATTACCACTGAACTCCATTAG" was inserted into the BsaI site of pKLC21, and "pKLC21 BsaI stx1_488 vector" and "pKLC21 BsaI stx2_488 vector" were produced. By using these, the toxin genes stx1 and stx2 were detected by the same method as shown in FIG. 14.

### (Differentiation of carbapenem resistance gene by antibacterial phage in clinical isolate)

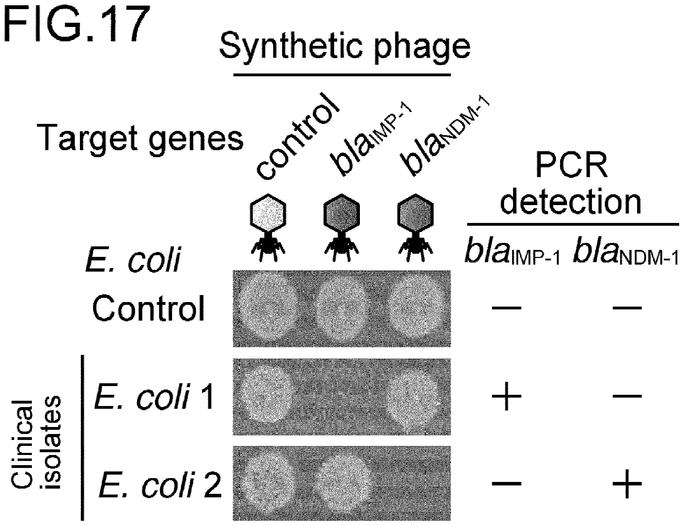

FIG. 17 is a photograph in which the carbapenem resistance genes bla_{IMP-1} and bla_{NDM-1} were identified to clinical isolates (E. coli1 and E. coli2) infected with antibacterial phage. These clinical isolates were obtained and maintained from an unspecified patient at Jichi Medical University.

The expression of bla_{IMP-1} and bla_{NDM-1} of the clinical isolates (E. coli1 and E. coli2) was confirmed by PCR reaction by using primers, IMP type det 255-f, "GGCGTTTATGTTCATACTTCG" and IMP type det 255-r, "AGATGCATACGTGGGGATAG", and NDM type det 209-f, "CCAATGCGTTGTCGAACCAG" and NDM type det 209-r, "GATCAGGCAGCCACCAAAAG".

### (Therapeutic effect of antibacterial phage on moth larvae infected with clinically isolated E. coli strain)

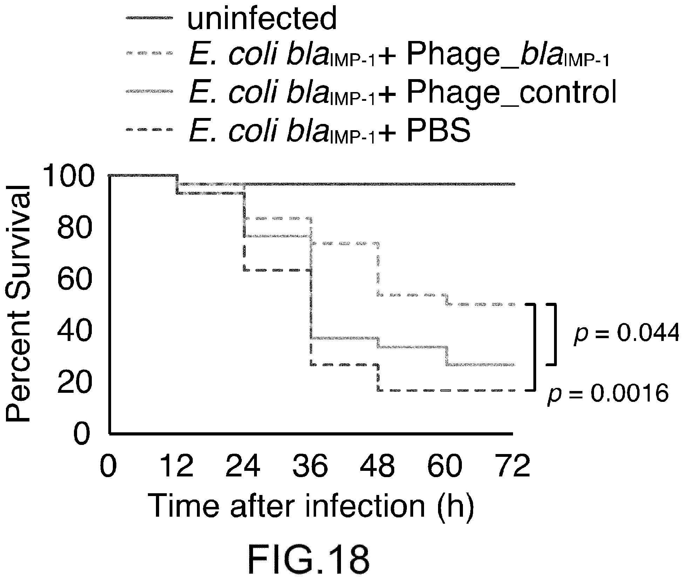

FIG. 18 is a graph examining the therapeutic effect of antibacterial phage on moth larvae infected with a clinically isolated E. coli strain.

M-sized larvae of Galleria mellonella were purchased from the "Live Bait Factory, inc." and used for the survival assay. After obtaining, they were acclimatized to the laboratory environment in the dark for 24 hours and then used for assay within 48 hours. In addition, larvae that were weak in movement, dark in color, unusual in shape, and clearly different in size from other larvae were not used in the experiment. The syringe used was a combination of a Hamilton syringe, leur tip (701LT, Hamilton) and a KF731 needle (Hamilton). E. coli cultured at 37 degree C in LB medium overnight was added to LB medium in an amount of 1/100 and shake-cultured at 37 degree C until OD600 reached about 0.5. It was then washed twice with PBS to make a solution of ~ 1 x 10⁷ CPU / ml. Twenty cream-colored larvae were taken, and 5 micro-l of PBS or bacterial solution was injected into the left proleg. One hour later, 5 micro-l each of SM buffer or antibacterial phage was injected into the right proleg. After that, the larvae were moved to an incubator at 37 degree C, and the progress was observed every 12 hours. Larvae that stopped responding to touch were determined to be dead.

According to the graph of FIG. 18, 80% of the larvae to which the clinically isolated E. coli expressing bla_{IMP-1} was administered died after 48 hours. On the other hand, the mortality rate in the group to which the antibacterial phage targeting bla_{IMP-1} was administered was about 50%, and there was a statistically significant difference in the mortality rate between the two groups. From this result, it was confirmed that the prepared antibacterial phage targeting bla_{IMP-1} has a therapeutic effect on bacterial infections in an experimental system in vivo.

### (Identification of Staphylococcus aureus methicillin resistance gene by antibacterial phage)

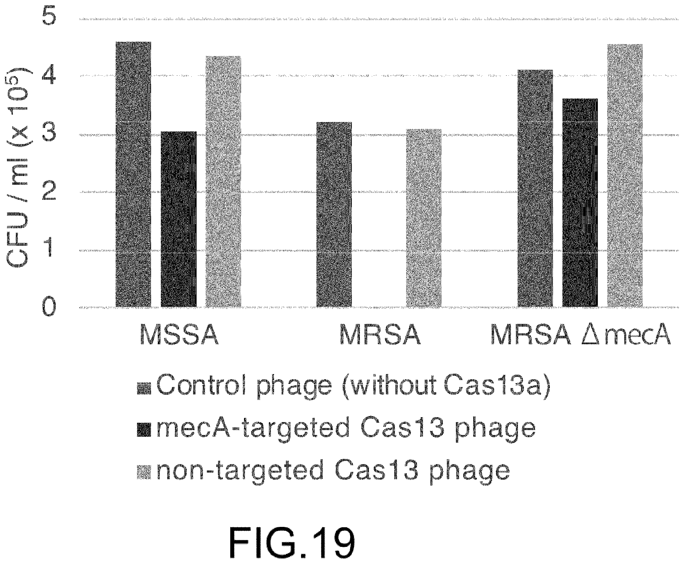

FIG. 19 is a graph of experimental results showing that antibacterial phage prepared for Staphylococcus aureus also has a selective bactericidal effect to the Staphylococcus aureus having the methicillin resistance gene mecA. The mecA crRNA-446-as, "TATCCCAAAGCATACATATTGAAAATTTAAAAT" and mecA crRNA-446-s, "AAACATTTTAAATTTTCAATATGTATGCTTTGG", which targeted mecA, were synthesized, after annealing, it was inserted into the BsaI site of pKLC21. The packaging sequences were ligated so that the Cas13a crRNA region of pKLC21 BsaI mecA was packaged in phage NM1, and Staphylococcus aureus RN4220 where NM1 phage lacking the packaging sequence was in a state of lysogenization was transformed by using it. Then, the transformed RN4220 strain was cultured in TSB medium at 37 degree C, and mitomycin C (2 micro-g / ml) was added when the OD600 reached 0.2. Then, the mixture was shaken at 30 degree C overnight at 80 rpm to obtain antibacterial phage through a 0.22 micro-m filter. For RN4220 that does not have mecA, clinical isolate USA300 that has mecA, and clinical isolate USA300-delta-mecA that lacks mecA, antibacterial phage targeting mecA was added in the same manner as in FIG. 14, and after culturing at 37 degree C for 12 hours, the resistance gene mecA was identified.

According to the results of FIG. 19, colony formation was not confirmed by antibacterial phage in USA300 having mecA, whereas colony formation was confirmed from Staphylococcus aureus strains (RN4220 and USA300-delta-mecA) not carrying mecA. From the above results, it was confirmed that not only Gram-negative E. coli but also Gram-positive Staphylococcus aureus can be tested for specific genes by the antibacterial phage.

## Claims

1. An antibacterial phage including: CRISPR-Cas13a having a target sequence that recognizes a specific gene as a target, preferably the target sequence is designed as a spacer sequence for crRNA of 14 to 28 bases,
wherein the specific gene is a drug resistance gene or a pathogenic gene included in the genome and/or plasmid of a bacterium of one or any combination of: Methicillin-Resistant Staphylococcus aureus (MRSA), Vancomycin-Resistant Staphylococcus aureus (VRSA), Vancomycin-Resistant enterococci (VRE), Penicillin-Resistant Streptococcus Pneumoniae (PRSP), Multidrug-resistant Pseudomonas aeruginosa (MDRP), Multiple Drug-Resistant Acinetobacter (MDRA), carbapenem-resistant Pseudomonas aeruginosa (CRPA), carbapenem-resistant Serratia (CRSA), third-generation cephalosporin-resistant pneumoniae (3GCRKP), third-generation cephalosporin-resistant E. coli (3GCREC), fluoroquinolone-resistant E. coli (FQREC), colistin-resistant E. coli (CoIR-EC),or
wherein the specific gene is a toxin gene or a pathogenic gene included in a bacterial genome and/or a plasmid of one or any combination of: Staphylococcus aureus, Clostridium botulinum, Vibrio cholerae, Escherichia coli, Vibrio parahaemolyticus, Clostridium tetani, Clostridium perfringens, Streptococcus pyogenes, Clostridium difficile, Bordetella pertussis, Corynebacterium diphtheriae, Shigella dysenteriae, Bacillus anthracis, Pseudomonas aeruginosa, Listeria monocytogenes, and Streptococcus pneumoniae.

2. The antibacterial phage of claim 1, wherein the drug resistance gene or pathogenic gene is one or any combination of: mecA, vanA, vanB, vanC, vanD, vanE, vanG, vanL, vanM, vanN, pbp1a, pbp2b, pbp2x, blaIMP, blaVIM, blaOXA, blaSHV, blaSME, blaNDM, blalMI, blaTEM, blaCMY, blaGES, blaCTX-M, blaKPC, aac(6'), parC, gyrA, qnrA, mcr, and mutation thereof.

3. The antibacterial phage according to claim 1, wherein the toxin gene includes one or any combination of: enterotoxin, botulinum neurotoxin, cholera toxin, heat-resistant enterotoxin, heat-labile enterotoxin, thermostable direct hemolysin, Shiga toxin, tetanus toxin, alpha toxin, leucocidin, beta toxin, toxic shock syndrome toxin, streptidine O, erythrogenic toxin, alpha hemolytic toxins, cytotoxic necrotizing factors I, toxin A, toxin B, pertussis toxin, diphtheria toxin, adhesin, secretion (permeation) apparatus, lysates, and gene for producing superantigens.

4. The antibacterial phage according to any one of claims 1 to 3, wherein the target sequence is a specific 14 - 28 base sequence of the target gene sequence.

5. The antibacterial phage according to any one of claims 1 to 4, wherein the target sequence includes: SEQ ID NO: 7, SEQ ID NO: 17, SEQ ID NO: 22, SEQ ID NO: 26, SEQ ID NO: 27, SEQ ID NO: 28, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, and SEQ ID NO: 52.

6. The antibacterial phage according to any one of claims 1 to 5, wherein the specific gene causes drug resistance by nucleic acid acquisition and/or nucleic acid mutation.

7. The antibacterial phage according to any one of claims 1 to 6, wherein the target is a plurality of sequences, and each of a plurality of specific genes is recognized as the target.

8. A therapeutic composition, bactericidal agent, or a bacteria identification kit including the antibacterial phage according to any one of claims 1 to 7.

9. A therapeutic composition manufacturing method comprising the steps of:
(i) transforming and/or infecting a phagemid having a packaging sequence, Cas13a, and CRISPR with a target sequence that recognizes a specific gene as defined in any one of claims 1 to 5 as a target, and helper phage with a phage-synthesizing bacteria, and lysing and/or releasing out of the cell; and
(ii) obtaining constructed antibacterial phage.

10. An antibacterial phage including CRISPR-Cas13a for use in a bacteria elimination method comprising the steps of:
(i) applying antibacterial phage including CRISPR-Cas13a having a target sequence that recognizes a specific gene as defined in any one of claims 1 to 5 as a target; and
(ii) eliminating bacteria having the specific gene, preferably the bacteria are present in bacterial flora of humans and/or animals.

11. An ex *vivo* bacteria elimination method comprising the steps of:
(i) applying antibacterial phage including CRISPR-Cas13a having a target sequence that recognizes a specific gene as defined in any one of claims 1 to 5 as a target; and
(ii) eliminating bacteria having the specific gene, preferably the bacteria are present in an environment and/or food.

12. An antibacterial phage including CRISPR-Cas13a for use in a bacteria identification method comprising the steps of:
(i) infecting bacteria with antibacterial phage including CRISPR-Cas13a having a target sequence that recognizes a specific gene as a target; and
(ii) determining and/or testing whether the bacteria have the specific gene.

13. An ex *vivo* bacteria identification method comprising the steps of:
(i) infecting bacteria with antibacterial phage including CRISPR-Cas13a having a target sequence that recognizes a specific gene as a target; and
(ii) determining and/or testing whether the bacteria have the specific gene.

14. The antibacterial phage including CRISPR-Cas13a for use in the bacteria identification method according to claim 12, or the bacteria identification method according to claim 13, wherein the antibacterial phage includes a sequence of an antibiotic resistance gene.

15. The antibacterial phage including CRISPR-Cas13a for use in the bacteria identification method according to claim 12, wherein the bacteria are present in the bacterial flora in humans or animals; or the bacteria identification method according to claim 13, wherein the bacteria are present in foods or the environment, and/or are genetically modified bacteria.

16. An antibacterial phage according to any of claims 1-7 for use in treating infectious diseases of the animal other than a human that is caused by bacteria having the specific gene.

## Patentansprüche

1. Ein antibakterieller Phage, enthaltend: CRISPR-Cas13a mit einer Zielsequenz, welche ein spezifisches Gen als Ziel erkennt, wobei die Zielsequenz vorzugsweise als eine Spacer-Sequenz für crRNA mit 14 bis 28 Basen gestaltet ist,
wobei das spezifische Gen ein Arzneimittelresistenzgen oder ein pathogenes Gen ist, welches in dem Genom und/oder Plasmid eines Bakteriums enthalten ist von einem oder einer beliebigen Kombination von: Methicillin-resistenter Staphylococcus aureus (MRSA), Vancomycin-resistenter Staphylococcus aureus (VRSA), Vancomycinresistente Enterokokken (VRE), Penicillin-resistenter Streptococcus pneumoniae (PRSP), multiresistenter Pseudomonas aeruginosa (MDRP), Mehrfacharzneimittelresistenter Acinetobacter (MDRA), Carbapenem-resistenter Pseudomonas aeruginosa (CRPA), Carbapenem-resistenter Serratia (CRSA), Cephalosporin-resistenter Pneumoniae der dritten Generation (3GCRKP), Cephalosporin-resistenter E. coli der dritten Generation (3GCREC), Fluorchinolon-resistenter E. coli (FQREC), Colistin-resistenter E. coli (CoIR-EC), oder
wobei das spezifische Gen ein Toxin-Gen oder ein pathogenes Gen ist, welches in einem bakteriellen Genom und/oder einem Plasmid enthalten ist von einem oder einer beliebigen Kombination von: Staphylococcus aureus, Clostridium botulinum, Vibrio cholerae, Escherichia coli, Vibrio parahaemolyticus, Clostridium tetani, Clostridium perfringens, Streptococcus pyogenes, Clostridium difficile, Bordetella pertussis, Corynebacterium diphtheriae, Shigella dysenteriae, Bacillus anthracis, Pseudomonas aeruginosa, Listeria monocytogenes und Streptococcus pneumoniae.

2. Der antibakterielle Phage nach Anspruch 1, wobei das Medikamentenresistenzgen oder das pathogene Gen eines oder eine beliebige Kombination ist von: mecA, vanA, vanB, vanC, vanD, vanE, vanG, vanL, vanM, vanN, pbp1a, pbp2b, pbp2x, bIaIMP, blaVIM, blaOXA, blaSHV, blaSME, blaNDM, blaIMI, blaTEM, blaCMY, blaGES, blaCTX-M, blaKPC, aac(6'), parC, gyrA, qnrA, mcr, und Mutationen davon.

3. Der antibakterielle Phage nach Anspruch 1, wobei das Toxin-Gen eines oder eine beliebige Kombination enthält von: Enterotoxin, Botulinum-Neurotoxin, Cholera-Toxin, hitzeresistentes Enterotoxin, hitzelabiles Enterotoxin, thermostabiles direktes Hämolysin, Shiga-Toxin, Tetanus-Toxin, Alpha-Toxin, Leucocidin, Beta-Toxin, Toxic Shock Syndrome-Toxin, Streptidin O, erythrogenes Toxin, hämolytische Alpha-Toxine, zytotoxische nekrotisierende Faktoren I, Toxin A, Toxin B, Pertussis-Toxin, Diphtherie-Toxin, Adhäsin, Sekretions-(Permeations-)Apparat, Lysate, und Gen zur Herstellung von Superantigenen.

4. Der antibakterielle Phage nach einem der Ansprüche 1 bis 3, wobei die Zielsequenz eine spezifische 14-28 Basensequenz der Zielgensequenz ist.

5. Der antibakterielle Phage nach einem der Ansprüche 1 bis 4, wobei die Zielsequenz enthält: SEQ ID NR: 7, SEQ ID NR: 17, SEQ ID NR: 22, SEQ ID NR: 26, SEQ ID NR: 27, SEQ ID NR: 28, SEQ ID NR: 36, SEQ ID NR: 37, SEQ ID NR: 38, SEQ ID NR: 39, SEQ ID NR: 40, SEQ ID NR: 41, SEQ ID NR: 42, SEQ ID NR: 43, SEQ ID NR: 44, SEQ ID NR: 45, SEQ ID NR: 46, SEQ ID NR: 47, SEQ ID NR: 48, SEQ ID NR: 49, SEQ ID NR: 50, SEQ ID NR: 51, und SEQ ID NR: 52.

6. Der antibakterielle Phage nach einem der Ansprüche 1 bis 5, wobei das spezifische Gen eine Arzneimittelresistenz durch Nukleinsäureerwerb und/oder Nukleinsäuremutation verursacht.

7. Der antibakterielle Phage nach einem der Ansprüche 1 bis 6, wobei das Ziel eine Vielzahl von Sequenzen ist und jedes Gen einer Vielzahl von spezifischen Genen als Ziel erkannt wird.

8. Eine therapeutische Zusammensetzung, bakterizides Mittel, oder ein Bakterien-Identifikationskit, enthaltend den antibakteriellen Phagen nach einem der Ansprüche 1 bis 7.

9. Ein Verfahren zur Herstellung einer therapeutischen Zusammensetzung, umfassend die Schritte:
(i) Transformieren und/oder Infizieren eines Phagemids mit einer Verpackungssequenz, Cas13a und CRISPR mit einer Zielsequenz, welche ein spezifisches Gen wie es in einem der Ansprüche 1 bis 5 definiert ist, als Ziel erkennt, und eines Helfer-Phagen mit einem Phagen-synthetisierenden Bakterium, und Lysieren und/oder Freisetzen aus der Zelle; und
(ii) Erhalten eines konstruierten antibakteriellen Phagen.

10. Ein antibakterieller Phage, enthaltend CRISPR-Cas13a, zur Verwendung in einem Bakterien-Eliminierungsverfahren, umfassend die Schritte:
(i) Anwenden eines antibakteriellen Phagen, enthaltend CRISPR-Cas13a mit einer Zielsequenz, welche ein spezifisches Gen wie in einem der Ansprüche 1 bis 5 definiert, als Ziel erkennt; und
(ii) Eliminieren von Bakterien mit dem spezifischen Gen, wobei die Bakterien vorzugsweise in der Bakterienflora von Menschen und/oder Tieren vorhanden sind.

11. Ein *ex-vivo* Bakterien-Eliminierungsverfahren, umfassend die Schritte:
(i) Anwenden eines antibakteriellen Phagen, enthaltend CRISPR-Cas13a mit einer Zielsequenz, welche ein spezifisches Gen wie in einem der Ansprüche 1 bis 5 definiert, als Ziel erkennt; und
(ii) Eliminieren von Bakterien mit dem spezifischen Gen, wobei die Bakterien vorzugsweise in der Umwelt und/oder in Lebensmitteln vorhanden sind.

12. Ein antibakterieller Phage, enthaltend CRISPR-Cas13a, zur Verwendung in einem Bakterien-Identifikationsverfahren, umfassend die Schritte:
(i) Infizieren von Bakterien mit einem antibakteriellem Phagen, enthaltend CRISPR-Cas13a mit einer Zielsequenz, die ein spezifisches Gen als Ziel erkennt; und
(ii) Bestimmen und/oder Prüfen, ob die Bakterien das spezifische Gen haben.

13. Ein *ex-vivo* Bakterien-Identifikationsverfahren, umfassend die Schritte:
(i) Infizieren von Bakterien mit einem antibakteriellem Phagen, enthaltend CRISPR-Cas13a mit einer Zielsequenz, die ein spezifisches Gen als Ziel erkennt; und
(ii) Bestimmen und/oder Prüfen, ob die Bakterien das spezifische Gen haben.

14. Der antibakterielle Phage, enthaltend CRISPR-Cas13a, zur Verwendung in dem Bakterien-Identifikationsverfahren nach Anspruch 12 oder dem Bakterien-identifikationsverfahren nach Anspruch 13, wobei der antibakterielle Phage eine Sequenz eines Antibiotikaresistenzgens enthält.

15. Der antibakterielle Phage, enthaltend CRISPR-Cas13a, zur Verwendung in dem Bakterien-Identifikationsverfahren nach Anspruch 12, wobei die Bakterien in der Bakterienflora von Menschen oder Tieren vorhanden sind; oder die Bakterienidentifizierungsmethode nach Anspruch 13, wobei die Bakterien in Nahrungsmitteln oder in der Umwelt vorhanden sind und/oder genetisch veränderte Bakterien sind.

16. Ein antibakterieller Phage nach einem der Ansprüche 1-7 zur Verwendung bei der Behandlung von Infektionskrankheiten eines Tieres, das kein Mensch ist, welche durch Bakterien mit dem spezifischen Gen verursacht werden.

## Revendications

1. Phage antibactérien incluant: CRISPR-Cas13a comportant une séquence cible qui reconnaît un gène spécifique en tant que cible, de préférence la séquence cible est conçue sous la forme d'une séquence d'espaceur pour un ARNcr de 14 à 28 bases,
dans lequel le gène spécifique est un gène de pharmacorésistance ou un gène pathogène inclus dans le génome et/ou le plasmide d'une bactérie choisie parmi une bactérie ou toute combinaison de bactéries parmi : Staphylococcus aureus résistante à la méthicilline (SARM), Staphylococcus aureus résistante à la vancomycine (SARV), entérocoques résistants à la vancomycine (ERV), Streptococcus pneumoniae résistante à la pénicilline (SPRP), Pseudomonas aeruginosa multirésistante (PAMR), Acinetobacter multirésistante (AMR), Pseudomonas aeruginosa résistante aux carbapénèmes (PARC), Serratia résistante aux carbapénèmes (SRC), pneumoniae résistante aux céphalosporines de troisième génération (3GCRKP), E. coli résistante aux céphalosporines de troisième génération (3GCREC), E. coli résistante aux fluoroquinolones (FQREC), E. coli résistante à la colistine (CoIR-EC), ou
dans lequel le gène spécifique est un gène de toxine ou un gène pathogène inclus dans un génome bactérien et/ou un plasmide d'une bactérie ou de toute combinaison de bactéries parmi : Staphylococcus aureus, Clostridium botulinum, Vibrio cholerae, Escherichia coli, Vibrio parahaemolyticus, Clostridium tetani, Clostridium perfringens, Streptococcus pyogenes, Clostridium difficile, Bordetella pertussis, Corynebacterium diphtheriae, Shigella dysenteriae, Bacillus anthracis, Pseudomonas aeruginosa, Listeria monocytogenes, et Streptococcus pneumoniae.

2. Phage antibactérien selon la revendication 1, dans lequel le gène de pharmacorésistance ou le gène pathogène est un gène ou toute combinaison de gènes parmi : mecA, vanA, vanB, vanC, vanD, vanE, vanG, vanL, vanM, vanN, pbp1a, pbp2b, pbp2x, blalMP, blaVIM, blaOXA, blaSHV, blaSME, blaNDM, blalMI, blaTEM, blaCMY, blaGES, blaCTX-M, blaKPC, aac(6'), parC, gyrA, qnrA, mcr, et une mutation de ceux-ci.

3. Phage antibactérien selon la revendication 1, dans lequel le gène de toxine comprend une ou toute combinaison de : entérotoxine, neurotoxine botulique, toxine cholérique, entérotoxine thermorésistante, entérotoxine thermolabile, hémolysine directe thermostable, shigatoxine, toxine tétanique, toxine alpha, leucocidine, toxine bêta, toxine du syndrome de choc toxique, streptidine O, toxine érythrogénique, toxines hémolytiques alpha, facteurs nécrosants cytotoxiques I, toxine A, toxine B, toxine pertussique, toxine diphtérique, adhésine, système de sécrétion (perméation), lysats, et gène pour la production de superantigènes.

4. Phage antibactérien selon l'une quelconque des revendications 1 à 3, dans lequel la séquence cible est une séquence spécifique de 14-28 bases de la séquence génique cible.

5. Phage antibactérien selon l'une quelconque des revendications 1 à 4, dans lequel la séquence cible comprend : SEQ ID NO: 7, SEQ ID NO: 17, SEQ ID NO: 22, SEQ ID NO: 26, SEQ ID NO: 27, SEQ ID NO: 28, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, et SEQ ID NO: 52.

6. Phage antibactérien selon l'une quelconque des revendications 1 à 5, dans lequel le gène spécifique provoque une pharmacorésistance par acquisition d'acide nucléique et/ou mutation d'acide nucléique.

7. Phage antibactérien selon l'une quelconque des revendications 1 à 6, dans lequel la cible est une pluralité de séquences, et chacun parmi une pluralité de gènes spécifiques est reconnu en tant que cible.

8. Composition thérapeutique, agent bactéricide, ou trousse d'identification de bactéries comprenant le phage antibactérien selon l'une quelconque des revendications 1 à 7.

9. Procédé de fabrication d'une composition thérapeutique, comprenant les étapes de :
(i) transformation et/ou infection d'un phagémide comportant une séquence d'empaquetage, Cas13a, et CRISPR avec une séquence cible qui reconnaît un gène spécifique tel que défini dans l'une quelconque des revendications 1 à 5 en tant que cible, et un phage helper avec des bactéries de synthèse de phage, et lyse et/ou relargage hors de la cellule ; et
(ii) obtention d'un phage antibactérien construit.

10. Phage antibactérien incluant CRISPR-Cas13a pour une utilisation dans un procédé d'élimination de bactéries comprenant les étapes de :
(i) application d'un phage antibactérien incluant CRISPR-Cas13a comportant une séquence cible qui reconnaît un gène spécifique tel que défini dans l'une quelconque des revendications 1 à 5 en tant que cible ; et
(ii) élimination des bactéries comportant le gène spécifique, de préférence les bactéries sont présentes dans la flore bactérienne d'humains et/ou d'animaux.

11. Procédé d'élimination de bactéries *ex vivo* comprenant les étapes de :
(i) application d'un phage antibactérien incluant CRISPR-Cas13a comportant une séquence cible qui reconnaît un gène spécifique tel que défini dans l'une quelconque des revendications 1 à 5 en tant que cible ; et
(ii) élimination des bactéries comportant le gène spécifique, de préférence les bactéries sont présentes dans un environnement et/ou un aliment.

12. Phage antibactérien incluant CRISPR-Cas13a pour une utilisation dans un procédé d'identification de bactéries comprenant les étapes de :
(i) infection de bactéries avec un phage antibactérien incluant CRISPR-Cas13a comportant une séquence cible qui reconnaît un gène spécifique en tant que cible ; et
(ii) détermination et/ou test si les bactéries comportent le gène spécifique.

13. Procédé d'identification de bactéries ex *vivo* comprenant les étapes de :
(i) infection de bactéries avec un phage antibactérien incluant CRISPR-Cas13a comportant une séquence cible qui reconnaît un gène spécifique en tant que cible ; et
(ii) détermination et/ou test si les bactéries comportent le gène spécifique.

14. Phage antibactérien incluant CRISPR-Cas13a pour une utilisation dans le procédé d'identification de bactéries selon la revendication 12, ou le procédé d'identification de bactéries selon la revendication 13, dans lequel le phage antibactérien inclut une séquence d'un gène d'antibiorésistance.

15. Phage antibactérien incluant CRISPR-Cas13a pour une utilisation dans le procédé d'identification de bactéries selon la revendication 12, dans lequel les bactéries sont présentes dans la flore bactérienne d'humains ou d'animaux ; ou le procédé d'identification de bactéries selon la revendication 13, dans lequel les bactéries sont présentes dans des aliments ou l'environnement, et/ou sont des bactéries génétiquement modifiées.

16. Phage antibactérien selon l'une quelconque des revendications 1 à 7 pour une utilisation dans le traitement de maladies infectieuses de l'animal autre qu'un humain qui sont causées par des bactéries comportant le gène spécifique.
